# EUROPEAN PATENT APPLICATION

(11) **EP 4 768 492 A1**
(43) Date of publication of application: **01.07.2026**
(21) Application number: 24870650.9
(22) Date of filing: 23.09.2024
(51) Int. Cl.: C07H 19/207, C07H 21/02, C07H 1/00, A61K 31/7088

(54) **CAPPING COMPOUND, AND APPLICATION THEREOF IN MRNA CAPPING**

(30) Priority: 26.09.2023 CN 202311243069
(71) Applicant: Jiangsu Synthgene Biotechnology Co., Ltd., Nanjing, Jiangsu 210000 (CN)
(72) Inventor: TONG, Kun, Nanjing, Jiangsu 210000 (CN)
(74) Representative: Arnold & Siedsma
(86) International application number: PCT/CN2024/120364
(87) International publication number: WO 2025/067106

(57) **Abstract**

The present invention provides a capping compound, and an application thereof in mRNA capping. The capping compound is a compound which conforms to the structure shown in the following formula, or a stereoisomer, pharmaceutically acceptable salt or solvate thereof. Since a 3 to 6 membered cycloalkyl group or a 3 to 6 membered heterocyclic group is provided, the capping compound of the present invention can improve mRNA stability, is not easily hydrolyzed by a decapping enzyme, and can improve an mRNA translation effect.

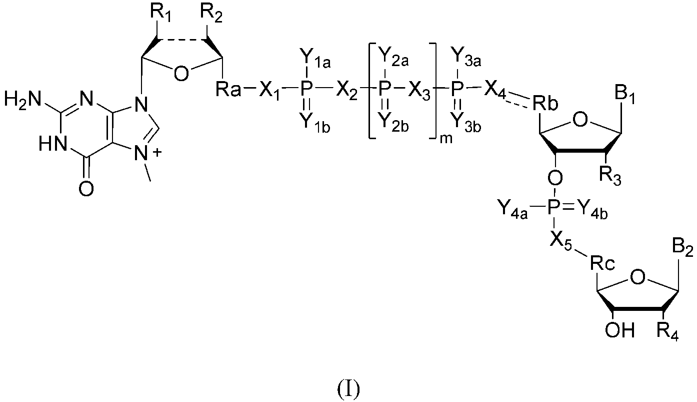

## Description

The present application claims priority to the Chinese Patent Application No. 202311243069.2 entitled "CAPPING COMPOUND, AND APPLICATION THEREOF IN MRNA CAPPING" and filed with the China National Intellectual Property Administration on September 26, 2023, which is incorporated herein by reference in its entirety.

### FIELD

The present disclosure relates to the technical field of biomedicine, and in particular to a capping compound and use thereof in mRNA capping.

### BACKGROUND

One of the main technical problems to be solved for mRNA is how to improve the stability and translation efficiency of mRNA. The 5'UTR and 3'UTR located on both sides of the protein coding sequence directly affect the translation efficiency and stability of mRNA and determine the effects of mRNA vaccines or drugs. Generally, by the addition of a 5' cap structure (Cap) and a polyadenylic acid Poly(A) tail structure, the stability of mRNA vaccines or drugs is enhanced, enabling them to maintain full biological activity for a relatively long time under different conditions.

Currently, the 5' cap structures on the market generally have disadvantages such as low capping efficiency and low mRNA translation efficiency. The present application develops a circular modified 5' cap structure, which can not only improve capping efficiency and mRNA translation efficiency at the cellular level, but also improve mRNA translation efficiency in mice.

### SUMMARY

In order to solve the problems of low capping efficiency and low mRNA translation efficiency of the existing capping analogs, the present application provides a capping compound and use thereof in mRNA capping.

The present disclosure provides a capping compound, which is a compound conforming to a structure represented by formula (I), or a stereoisomer, a pharmaceutically acceptable salt, or a solvate thereof, 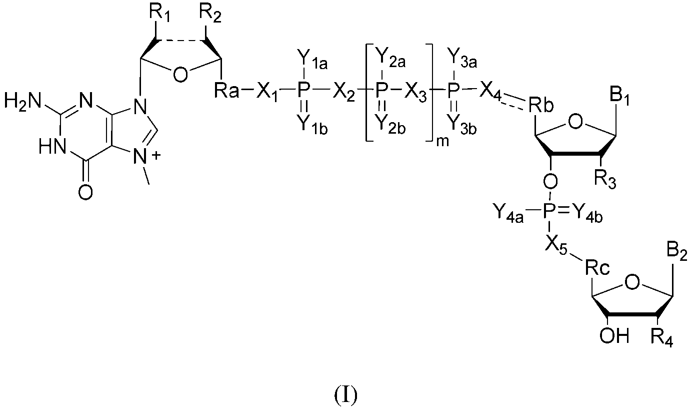 wherein
- - - represents a single bond or none;
-̅ -̅ -̅ represents a single bond or a double bond;
R₁ and R₂ are each independently H, OH, halogen, alkyl, alkoxy, alkenyl, alkynyl, cycloalkyl, cycloalkenyl, heterocyclyl, heteroaryl, alkylthio, or alkoxy forming a bridged ring with the carbon at 4', wherein the alkyl, alkoxy, alkenyl, alkynyl, cycloalkyl, cycloalkenyl, heterocyclyl, heteroaryl, and alkylthio are unsubstituted or substituted with one or more R₅, and R₅ is any one of alkyl, alkoxy, and substituted or unsubstituted acetamido;
R₃ is OH, halogen, alkoxy, or alkoxy forming a bridged ring with the carbon at 4', wherein the alkoxy is unsubstituted or substituted with one or more R₆, and R₆ is any one of alkyl and alkoxy;
R₄ is OH, alkyl, or alkoxy, wherein the alkyl and alkoxy are unsubstituted or substituted with one or more R₇, and R₇ is any one of alkyl and alkoxy;
Ra, Rb, and Rc are each independently 3- to 6-membered cycloalkyl, 3- to 6-membered heterocyclyl, CH₂, CH, or O, wherein Ra and X₁ are not simultaneously O, Rb and X₄ are not simultaneously O, and Rc and X₅ are not simultaneously O;
and at least one of Ra, Rb, and Rc is 3- to 6-membered cycloalkyl or 3- to 6-membered heterocyclyl;
X₁, X₂, X₃, X₄, and X₅ are each independently selected from O, S, CH₂, CH, and NH, or absent;
Y₁ₐ, Y₂ₐ, Y₃ₐ, and Y₄ₐ are each independently OH, SH, or BH₃;
Y_{1b}, Y_{2b}, Y_{3b}, and Y_{4b} are each independently O or S;
m is 0, 1, 2, or 3;
B₁ and B₂ are each independently a natural or modified nucleobase.

The pharmaceutically acceptable salt may be a salt well known to those skilled in the art, such as a sodium salt, a potassium salt, an ammonium salt, or an organic amine salt.

The capping analog of the above structural general formula can improve the stability of mRNA and is less susceptible to hydrolysis by decapping enzymes, thereby ultimately improving the translation efficiency of mRNA.

**In** an optional embodiment, one, two, or three of Ra, Rb, and Rc are 3- to 6-membered cycloalkyl or 3- to 6-membered heterocyclyl.

**In** a preferred embodiment, any one or two of Ra, Rb, and Rc are 3- to 6-membered cycloalkyl or 3- to 6-membered heterocyclyl.

Furthermore, any one or two of Ra, Rb, and Rc are three-membered or four-membered cycloalkyl having a structure selected from: 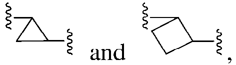 and X₁, X₄, or X₅ adjacent thereto is absent.

Preferably, the capping compound described in the present disclosure is a compound having a structure represented by formula (II), or a stereoisomer, a pharmaceutically acceptable salt, or a solvate thereof, 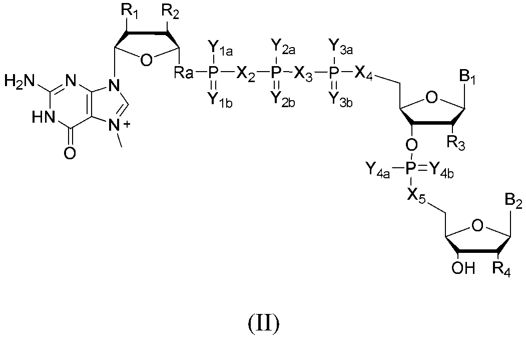 wherein Ra is selected from 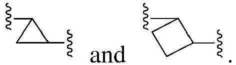 It exhibits excellent performance in capping efficiency and mRNA translation efficiency.

Further preferably, R₃ is H, OH, C₁₋₆ alkoxy, or LNA, wherein the C₁₋₆ alkoxy is unsubstituted or substituted with one or more R₆, and R₆ is C₁₋₆ alkyl or C₁₋₆ alkoxy.

Still further preferably, R₃ is H, OH, or C₁₋₄ alkoxy, and R₄ is H, OH, or C₁₋₄ alkoxy.

Furthermore, R₁ and R₂ are each independently H, OH, halogen, C₁₋₆ alkyl, C₁₋₆ alkoxy, or LNA, wherein the C₁₋₆ alkyl and C₁₋₆ alkoxy are unsubstituted or substituted with one or more R₅, and R₅ is any one of C₁₋₆ alkyl, C₁₋₆ alkoxy, and substituted or unsubstituted acetamido.

In the above preferred compound, the stereoisomer thereof, the pharmaceutically acceptable salt thereof, or the solvate thereof, R₁ and R₂ are each independently OH or C₁₋₄ alkoxy, wherein the C₁₋₄ alkoxy is unsubstituted or substituted with one or two R₅, and R₅ is C₁₋₄ alkyl or C₁₋₄ alkoxy.

Structurally, still further preferably, the capping compound described in the present disclosure is a compound having a structure represented by formula (III), or a stereoisomer, a pharmaceutically acceptable salt, or a solvate thereof,

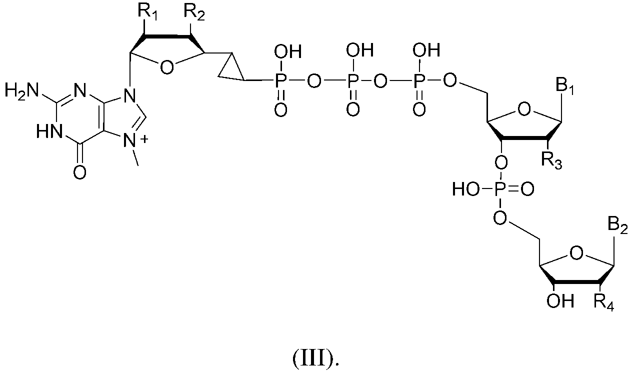

Further preferably, R₃ is selected from H, OH, -O-methyl, -O-ethyl, -O-propyl, -O-isopropyl, -O-methylene-O-methyl, -O-ethylene-O-methyl, -O-methylene-O-ethyl, -O-ethylene-O-ethyl, and LNA.

Further preferably, R₄ is hydroxyl or methoxy.

Further preferably, R₁ and R₂ are each independently selected from H, OH, F, Cl, -methylene-acetamido, methyl, ethyl, n-propyl, isopropyl, -O-methyl, -O-ethyl, -O-n-propyl, -O-isopropyl, -methylene-O-methyl, -ethylene-O-methyl, -methylene-O-ethyl, -ethylene-O-ethyl, -O-methylene-O-methyl, -O-ethylene-O-methyl, -O-methylene-O-ethyl, -O-ethylene-O-ethyl, -methylene-acetamido, -ethyl-acetamido, -n-propyl-acetamido, -isopropyl-acetamido, -n-butyl-acetamido, and -isobutyl-acetamido.

Further preferably, B₁ and B₂ are each independently adenine (A), guanine (G), cytosine (C), uracil (U), or thymine (T).

Furthermore, any one or two of Ra, Rb, and Rc may be three-membered or four-membered cycloalkyl having a structure selected from: 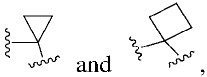 and X₁, X₄, or X₅ adjacent thereto is O.

Preferably, the capping compound described in the present disclosure is a compound having a structure represented by formula (IV), or a stereoisomer, a pharmaceutically acceptable salt, or a solvate thereof, 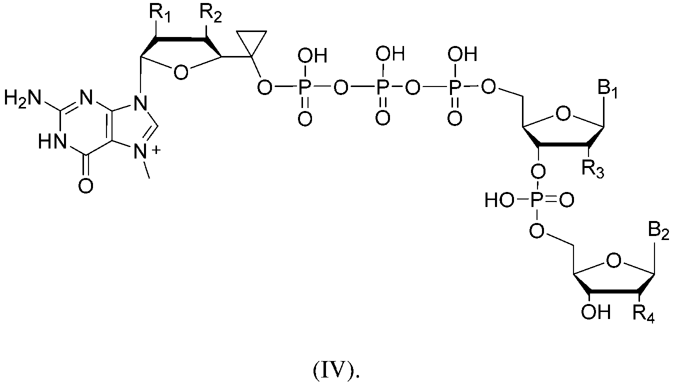
R₁ and R₂ are each independently OH or C₁₋₄ alkoxy, wherein the C₁₋₄ alkoxy is unsubstituted or substituted with one or more R₅, and R₅ is C₁₋₄ alkyl or C₁₋₄ alkoxy;
R₃ is C₁₋₆ alkoxy, wherein the C₁₋₆ alkoxy is unsubstituted or substituted with one or more R₆, and R₆ is C₁₋₆ alkyl or C₁₋₆ alkoxy;
R₄ is H, OH, or C₁₋₄ alkoxy.

Further preferably, R₃ is selected from H, OH, -O-methyl, -O-ethyl, -O-n-propyl, -O-isopropyl, -O-methylene-O-methyl, -O-ethylene-O-methyl, -O-methylene-O-ethyl, -O-ethylene-O-ethyl, and LNA.

Further preferably, R₄ is hydroxyl or methoxy.

Further preferably, R₁ and R₂ are each independently selected from H, OH, F, Cl, -methylene-acetamido, methyl, ethyl, n-propyl, isopropyl, -O-methyl, -O-ethyl, -O-n-propyl, -O-isopropyl, -methylene-O-methyl, -ethylene-O-methyl, -methylene-O-ethyl, -ethylene-O-ethyl, -O-methylene-O-methyl, -O-ethylene-O-methyl, -O-methylene-O-ethyl, -O-ethylene-O-ethyl, -methylene-acetamido, -ethyl-acetamido, -n-propyl-acetamido, -isopropyl-acetamido, -n-butyl-acetamido, and -isobutyl-acetamido.

Further preferably, B₁ and B₂ are each independently adenine (A), guanine (G), cytosine (C), uracil (U), or thymine (T).

The capping compound described in the present disclosure may be specifically selected from a compound represented by any one of the following, or a stereoisomer, a pharmaceutically acceptable salt, or a solvate thereof,

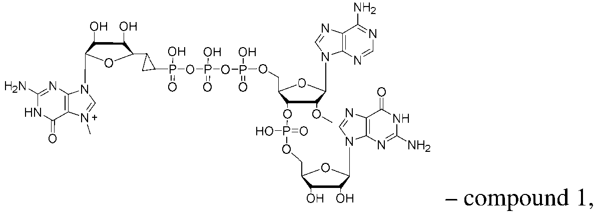

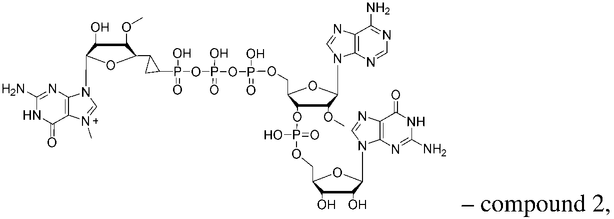

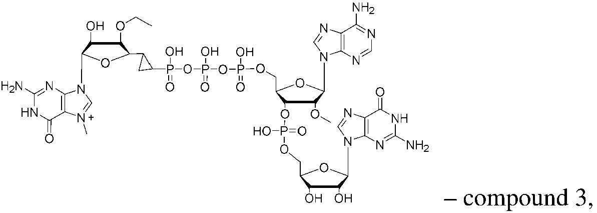

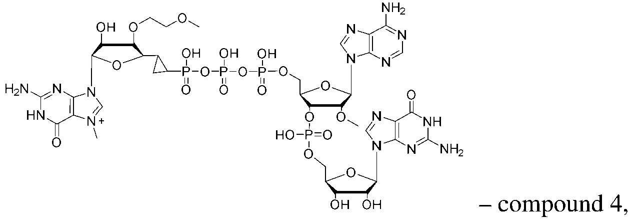

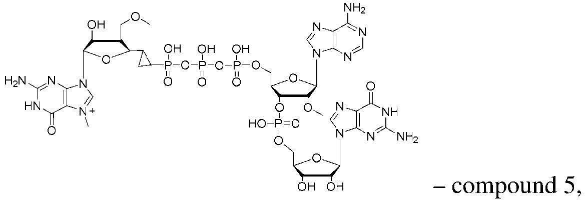

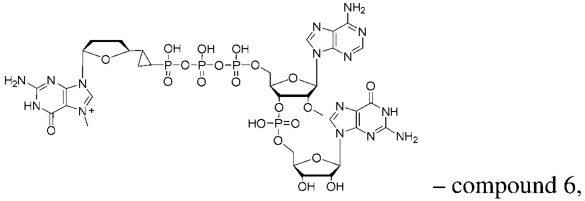

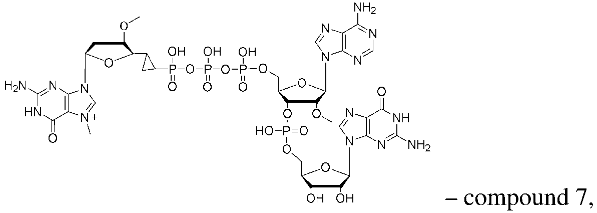

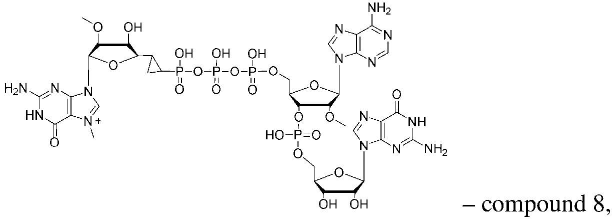

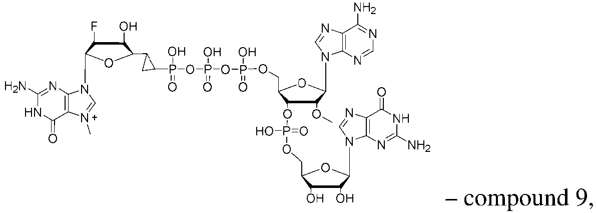

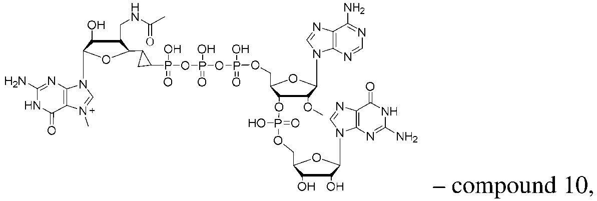

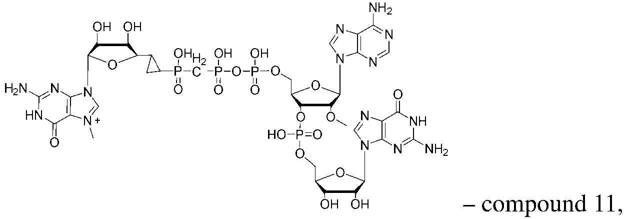

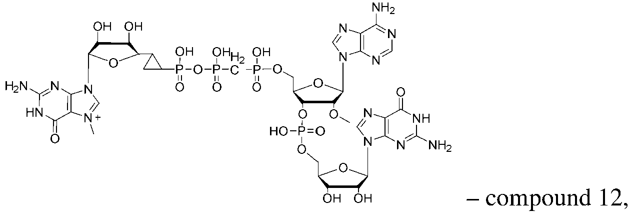

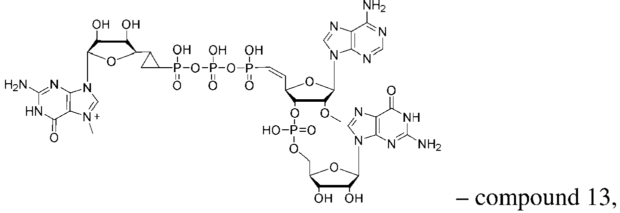

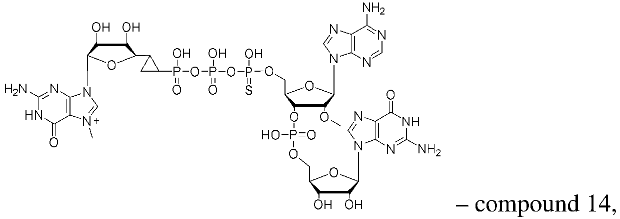

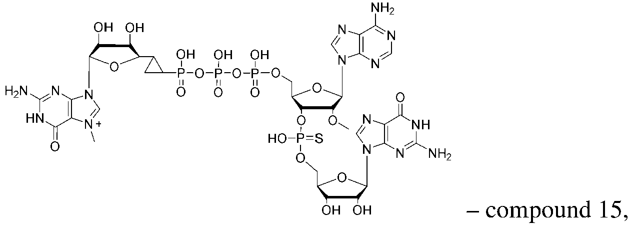

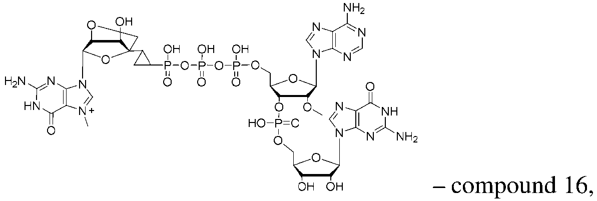

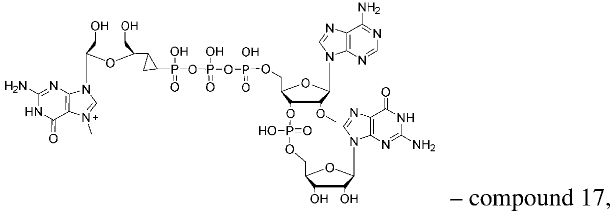

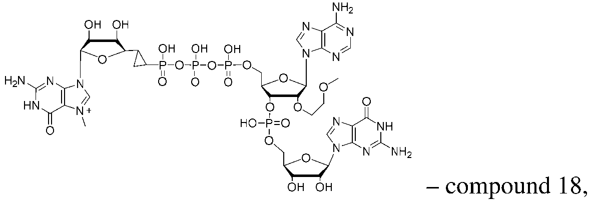

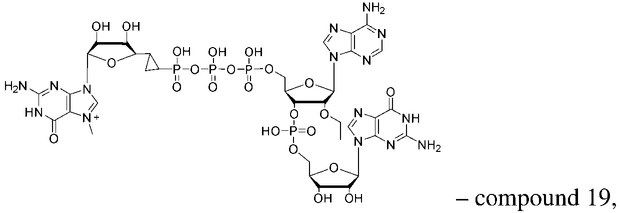

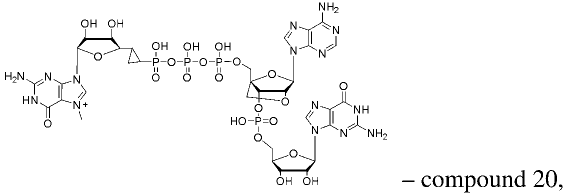

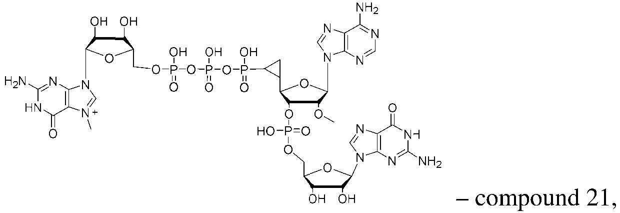

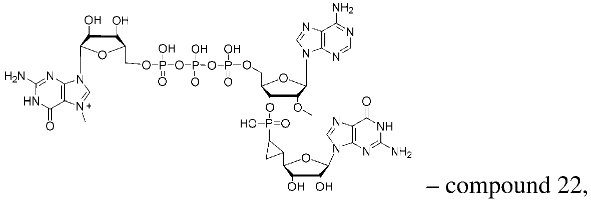

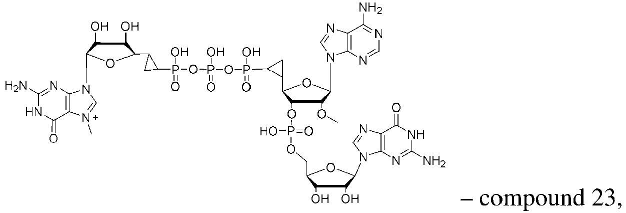

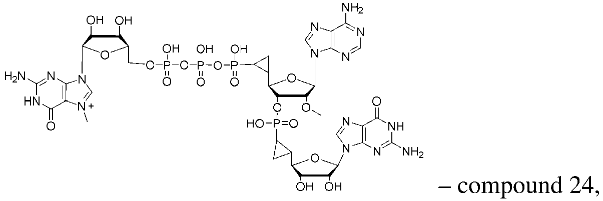

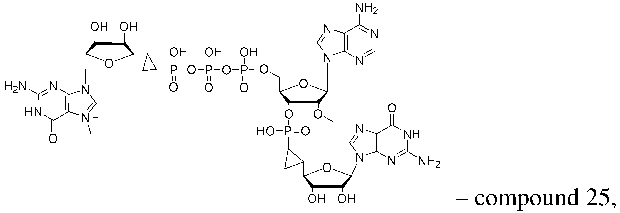

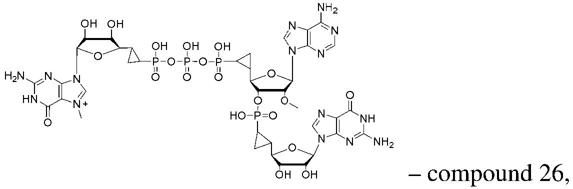

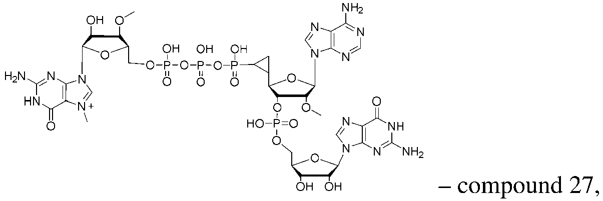

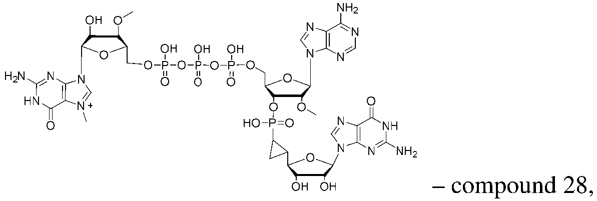

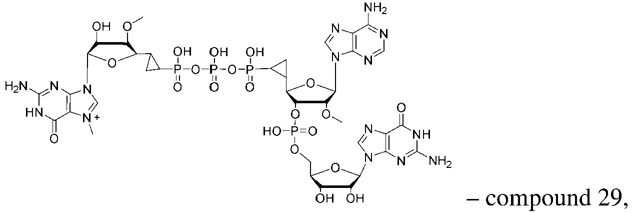

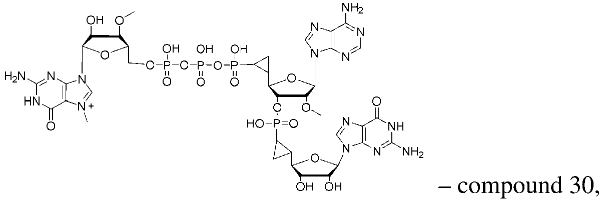

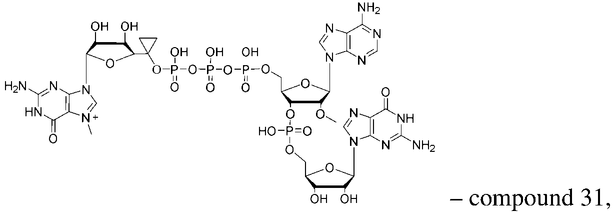

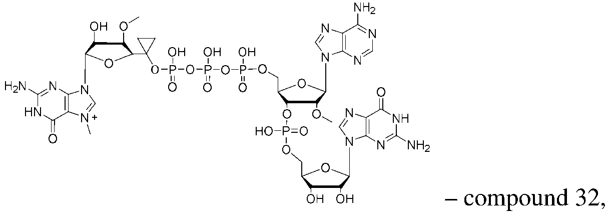

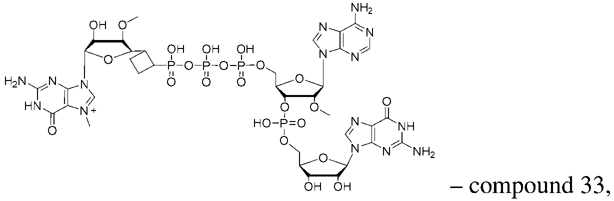

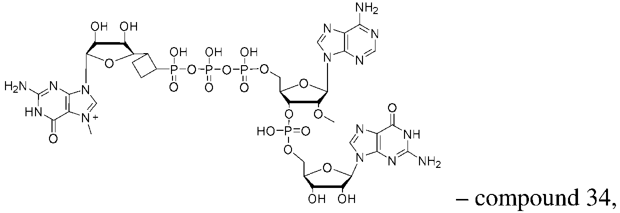

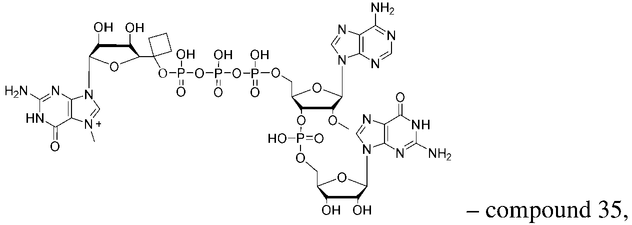

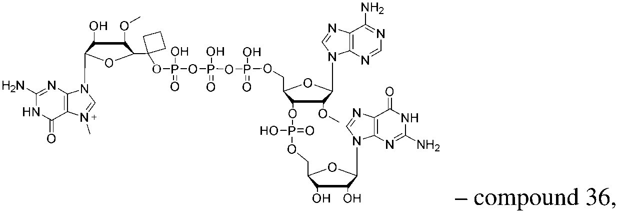

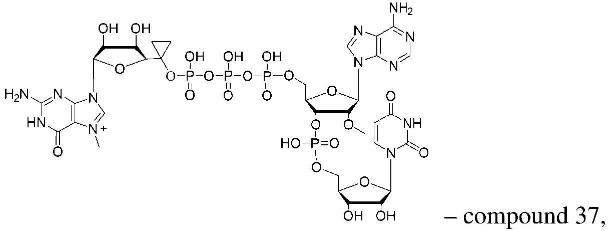

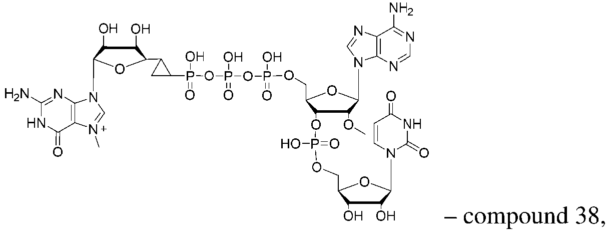

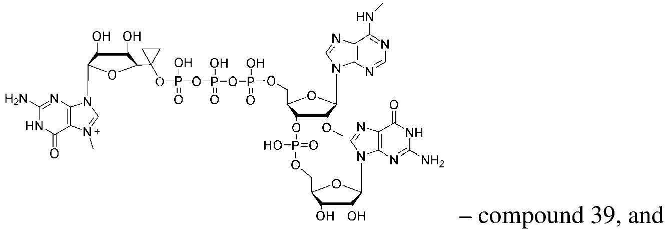

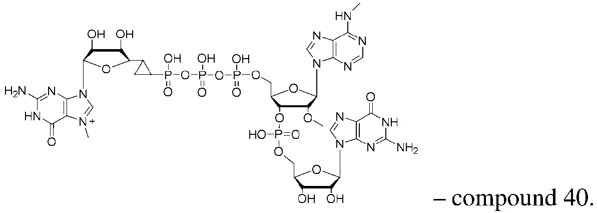

Alternatively, the following compounds may also serve as the capping compound for mRNA capping:

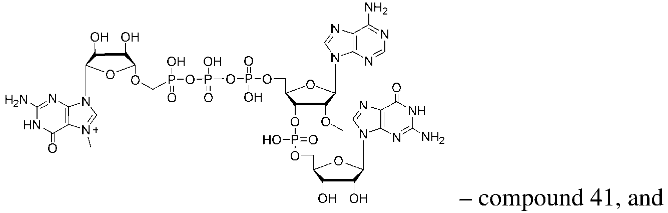

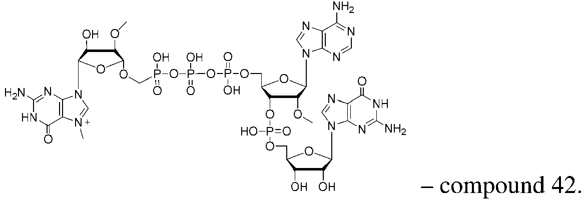

The capping compound described in the present disclosure, due to possessing three-membered to six-membered cycloalkyl or heterocyclyl, can improve the stability of mRNA and is less susceptible to hydrolysis by decapping enzymes, thereby ultimately improving the translation efficiency of mRNA.

Provided is use of the capping compound in the preparation of an *in vitro* co-transcriptional RNA capping reagent, wherein the structural fragment of the capping analog is present in mRNA to exert its effect, and is used for preparing a pharmaceutical composition.

The present disclosure further comprises a complex, comprising the capping compound described in the present disclosure, wherein the DNA template comprises a promoter region comprising a transcription start site, the transcription start site having a first nucleotide at nucleotide position +1 and a second nucleotide at nucleotide position +2; and B₁ is complementary to a nucleobase at position +1 of the DNA template, and B₂ is complementary to a nucleobase at transcription template position +2 on the DNA template.

The present disclosure further comprises an RNA molecule, having the capping compound described in the present disclosure.

The present disclosure further comprises a pharmaceutical composition, comprising the aforementioned RNA molecule and a pharmaceutically acceptable carrier.

### BRIEF DESCRIPTION OF DRAWINGS

FIG. 1: mRNA translation efficiency in mice for compounds 1, 2, 31, and 32.
FIG. 2: mRNA translation efficiency in mice for Comparative Example 1.

### DETAILED DESCRIPTION

Specific functional groups and chemical terms are defined below:
The term "alkyl" refers to a saturated aliphatic hydrocarbon group, which is a linear or branched group containing 1 to 20 carbon atoms, preferably alkyl containing 1 to 6 (e.g., 1, 2, 3, 4, 5, and 6) carbon atoms, and more preferably alkyl containing 1 to 4 carbon atoms.

The term "C₁₋₆ alkyl" refers to a linear or branched saturated hydrocarbon group having 1 to 6 carbon atoms. Examples of C₁₋₆ alkyl include: methyl, ethyl, n-propyl, isopropyl, n-butyl, tert-butyl, sec-butyl, isobutyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, etc.

The term "C₁₋₄ alkyl" refers to a linear or branched saturated hydrocarbon group having 1 to 4 carbon atoms. Examples of C₁₋₄ alkyl include: methyl, ethyl, n-propyl, isopropyl, n-butyl, tert-butyl, sec-butyl, isobutyl, etc.

The term "C₁₋₆ alkoxy" refers to an -O-R group, wherein R is as defined above for "C₁₋₆ alkyl".

The term "C₁₋₄ alkoxy" refers to an -O-R group, wherein R is as defined above for "C₁₋₄ alkyl".

"Halo" or "halogen" refers to fluorine (F), chlorine (Cl), bromine (Br), and iodine (I).

The term "cycloalkyl" includes any stable monocyclic or bicyclic ring having a specified number of carbon atoms, wherein any of the carbon atoms may be saturated or unsaturated. For example, 3- to 6-membered cycloalkyl is intended to include a monocyclic or bicyclic ring having 3, 4, 5, or 6 carbon atoms. Examples of 3- to 6-membered cycloalkyl include, but are not limited to, cyclopropyl, cyclobutyl, cyclobutenyl, cyclopentyl, cyclopentenyl, and cyclohexyl.

The term "3- to 6-membered heterocyclyl" refers to a saturated or unsaturated monocyclic ring comprising 3-6 ring atoms, wherein at least one of the ring atoms is selected from nitrogen, sulfur, and oxygen atoms.

"LNA", "locked nucleic acid unit", "locked nucleoside", and "locked nucleic acid" are used interchangeably, and include, but are not limited to, a methylene bridge between 2'O and 4'C of a nucleotide monomer, or refer to a sugar analog, a nucleoside, a nucleotide monomer, or a nucleic acid, each containing said bridge, for example, 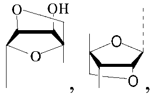 etc.

"Substituted" means that one or more, preferably up to 5, and more preferably 1 to 3, hydrogen atoms in the group are independently replaced by a corresponding number of substituents. It is self-evident that substituents are only located at their possible chemical positions, and those skilled in the art can determine (experimentally or theoretically) possible or impossible substitutions without undue effort.

In the present application, "base" and "nucleobase" are used interchangeably and may be either a natural nucleobase or a modified nucleobase. The natural nucleobase includes, but is not limited to, any one of adenine (A), guanine (G), cytosine (C), uracil (U), thymine (T), and derivatives thereof.

"Modified base" and "modified nucleobase" are used interchangeably and refer to a substance obtained by replacing one or two or more hydrogen atoms of a natural nucleobase, including, for example, but not limited to, N6-methyladenine, etc. In this embodiment, the modifying group is selected from N6-methyladenine, N1-methyladenine, N6-2'-O-dimethyladenosine, pseudouridine, N1-methylpseudouridine, 5-iodouridine, 4-thiouridine, 2-thiouridine, 5-methyluridine, pseudoisocytosine, 5-methoxycytosine, 2-thiocytosine, 5-hydroxycytosine, N1-methylcytosine, 5-hydroxymethylcytosine, hypoxanthine, N1-methylguanine, N1-methylguanine, and isoguanine.

"Stereoisomer" refers to compounds that have the same chemical constitution but differ in the spatial arrangement of atoms or groups. Stereoisomers include enantiomers, diastereoisomers, conformational isomers (rotamers), geometric isomers (cis/trans), atropisomers, etc.

### Example 1: Synthetic Route for Synthesis of Ammonium Salt of Compound 1 Using Intermediates A and B as Starting Materials

Intermediate A (15.0 mmol) and intermediate B (12.0 mmol) were suspended in DMF (90.0 mL), and ZnCl₂ (120.0 mmol) was added to the reaction solution under an ice bath. The reaction mixture was stirred at room temperature for 24 h, and then the reaction was quenched with a 0.25 M EDTA-2Na (144.0 mmol) solution. The mixture was loaded onto a DEAE Sephadex column. The product was eluted with a linear gradient of a 0-1.0 M aqueous ammonium bicarbonate eluent. Fractions with a purity of greater than 98% were collected, desalted by nanofiltration, and then concentrated to obtain the ammonium salt of compound 1. The reaction route is shown in the following equation:

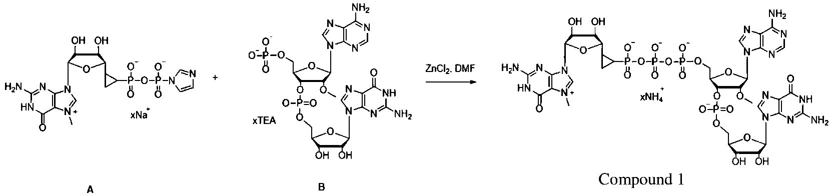

In the route, compound A was obtained by the following steps:
Intermediate A1 (0.86 mol) was dissolved in DMF (5.0 L), and DMSO (5.1 mol) and EDCI (2.6 mol) were added to the reaction solution before pyridine (0.86 mol) and trifluoroacetic acid (0.86 mol) were added dropwise. The reaction solution was allowed to react at room temperature for 5 h and then diluted with ethyl acetate. The organic phase was separately washed with a saturated aqueous sodium bicarbonate solution and water, then concentrated, and subjected to column chromatography to obtain intermediate A2.

Under a nitrogen atmosphere, tetraethyl methylenediphosphonate (0.78 mol) was added dropwise to a suspension of NaH (60%, 1.03 mol) in THF (1.0 L) under an ice bath, and then the mixture was stirred at 0 °C for 0.5 h. A solution of intermediate A2 (0.52 mol) in THF (2.0 L) was slowly added dropwise to the reaction solution. The mixture was stirred at room temperature overnight. The reaction was quenched with a saturated aqueous ammonium chloride solution under an ice bath, and the mixture was extracted three times with ethyl acetate (480.0 mL). The organic phases were combined, washed with saturated sodium chloride (300.0 mL × 2), concentrated under reduced pressure, and then purified by column chromatography to obtain intermediate A3.

Under a nitrogen atmosphere, trimethylsulfoxonium iodide (1.06 mol) was added to a suspension of NaH (60%, 1.88 mol) in DMSO (1.2 L) under an ice bath, and then the mixture was stirred at 0 °C for 1 h. A solution of intermediate A3 (0.47 mol) in DMSO (2.0 L) was slowly added dropwise to the reaction solution. The mixture was stirred at room temperature for 6 h. The reaction was quenched with a saturated aqueous ammonium chloride solution under an ice bath, and the mixture was extracted three times with ethyl acetate (450.0 mL). The organic phases were combined, washed with saturated sodium chloride (300.0 mL × 2), concentrated under reduced pressure, and then purified by column chromatography to obtain intermediate A4.

Intermediate A4 (0.21 mol) was dissolved in 1.5 L of acetonitrile, and triethylbromosilane (2.1 mol) was added to the reaction solution. The mixture was stirred at room temperature overnight and then concentrated to obtain a crude product of intermediate A5, which was directly used in the next step without purification.

The crude product of intermediate A5 was dissolved in THF (1.5 L), and TBAF (0.6 mol) was added at room temperature. The mixture was stirred at room temperature for 3 h and then concentrated under reduced pressure to obtain a crude product of intermediate A6, which was directly used in the next step without purification.

The crude product of intermediate A6 was dissolved in methanol (300.0 mL), and concentrated aqueous ammonia (1.3 L) was added to the reaction solution. The mixture was stirred at room temperature overnight. After the reaction was completed, the reaction solution was concentrated under reduced pressure and purified by reverse-phase chromatography to obtain intermediate A7.

Intermediate A7 (0.1 mol), triphenylphosphine (0.2 mol), 2,2'-dithiodipyridine (0.2 mol), imidazole (0.8 mol), and triethylamine (0.1 mol) were dissolved in DMF (380.0 mL), and the resulting solution was stirred for 10 h under a nitrogen atmosphere. After the reaction was completed, a 4 M solution of sodium perchlorate (0.4 mol) in acetone was added for precipitation. The mixture was filtered, and the filter cake was thoroughly washed with acetone to obtain intermediate A8.

Intermediate A8 (90.0 mmol) was dissolved in DMF (370.0 mL), and tributylamine phosphate (0.27 mol) and zinc chloride (0.72 mol) were added. The mixture was stirred at room temperature for 5 h. After the reaction was completed, the reaction solution was diluted with water, loaded onto DEAE Sephadex, and eluted with a linear gradient of a 0-1.0 M TEAB eluent. The eluate was concentrated to obtain intermediate A9.

Intermediate A9 (70.0 mmol) was dissolved in purified water (560.0 mL). The reaction solution was then cooled to 4 °C. Dimethyl sulfate (0.4 mol) was slowly added dropwise, and the pH was adjusted to no more than 5 with 2 M sodium hydroxide in the process. The reaction was monitored by HPLC. After the reaction was completed, the mixture was loaded onto DEAE Sephadex and eluted with a linear gradient of a 0-1.0 M TEAB eluent. The eluate was concentrated to obtain intermediate A10.

Intermediate A10 (53.0 mmol), triphenylphosphine (106.0 mmol), 2,2'-dithiodipyridine (106.0 mmol), imidazole (4.2 mol), and triethylamine (53.0 mmol) were dissolved in DMF (350.0 mL), and the resulting solution was stirred for 10 h under a nitrogen atmosphere. After the reaction was completed, a 4 M solution of sodium perchlorate (0.16 mol) in acetone was added for precipitation. The mixture was filtered, and the filter cake was thoroughly washed with acetone to obtain intermediate A.

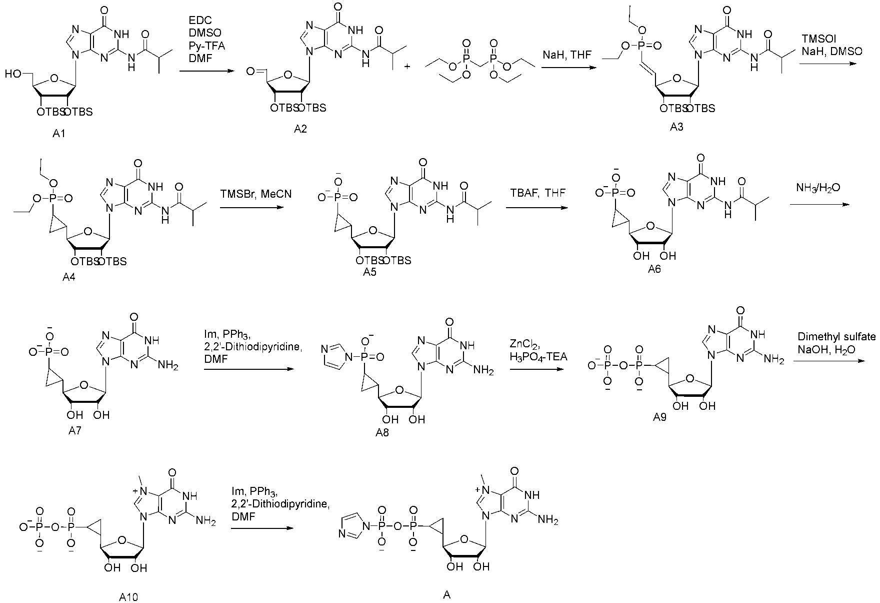

In the route, compound B was obtained by the following steps:
2'OMe-rA phosphoramidite monomer (0.23 mol) and N2-isobutyryl-2',3'-acetylguanosine (0.23 mol) were weighed and dissolved in DCM (2.0 L). Under a nitrogen atmosphere, tetrazole (0.5 mol) was added. The mixture was stirred at room temperature for 2 h. After the reaction was completed, an aqueous tert-butyl hydroperoxide solution (0.69 mol, 70%) was added dropwise to the reaction solution, and the mixture was allowed to react at room temperature for another 1 h. A solution of trichloroacetic acid (0.69 mol) in DCM (0.4 L) was added dropwise to the reaction solution, and the mixture was allowed to react at room temperature for 2 h. The reaction solution was separately washed with a 10% aqueous sodium sulfite solution, a 10% aqueous sodium bicarbonate solution, and saturated brine. The organic phase was concentrated under reduced pressure and then purified by column chromatography to obtain intermediate B1.

Intermediate B1 (0.18 mol) and bis(2-cyanoethyl)-N,N-diisopropylphosphoramidite (0.25 mol) were dissolved in DCM (1.7 L). Under a nitrogen atmosphere, tetrazole (0.39 mol) was added. The mixture was stirred at room temperature for 2 h. After the reaction was completed, an aqueous tert-butyl hydroperoxide solution (0.51 mol, 70%) was added dropwise to the reaction solution, and the mixture was allowed to react at room temperature for another 1 h. The reaction solution was separately washed with a 10% aqueous sodium sulfite solution, a 10% aqueous sodium bicarbonate solution, and saturated brine. The organic phase was concentrated under reduced pressure, and then the residue was dissolved in methanol (0.5 L) and aqueous ammonia (1.0 L). The resulting solution was stirred at room temperature overnight, concentrated to remove the solvent, then diluted with water, loaded onto DEAE Sephadex, and eluted with a linear gradient of a 0-1.0 M TEAB eluent. The eluate was concentrated to obtain intermediate B. Reaction route:

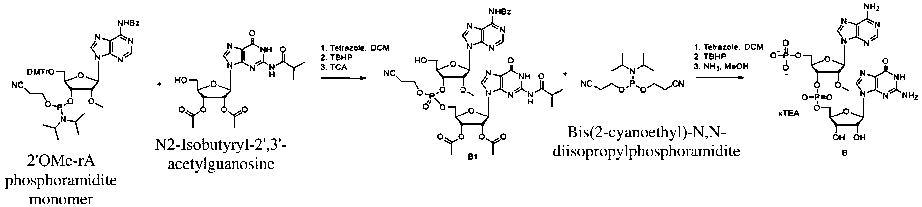

### Example 2: Synthetic Route for Synthesis of Ammonium Salt of Compound 2 Using Intermediates B and C as Starting Materials

With reference to the synthetic method for compound 1 in Example 1, the ammonium salt of compound 2 was obtained using intermediates B and C as starting materials. Reaction route:

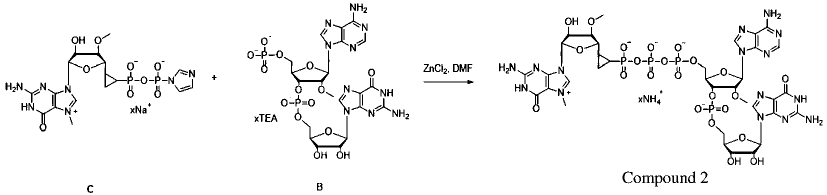

In the route, compound C was obtained by the following steps: With reference to the synthetic methods for intermediates A2-A in Example 1, intermediates C2-C were obtained. Reaction route:

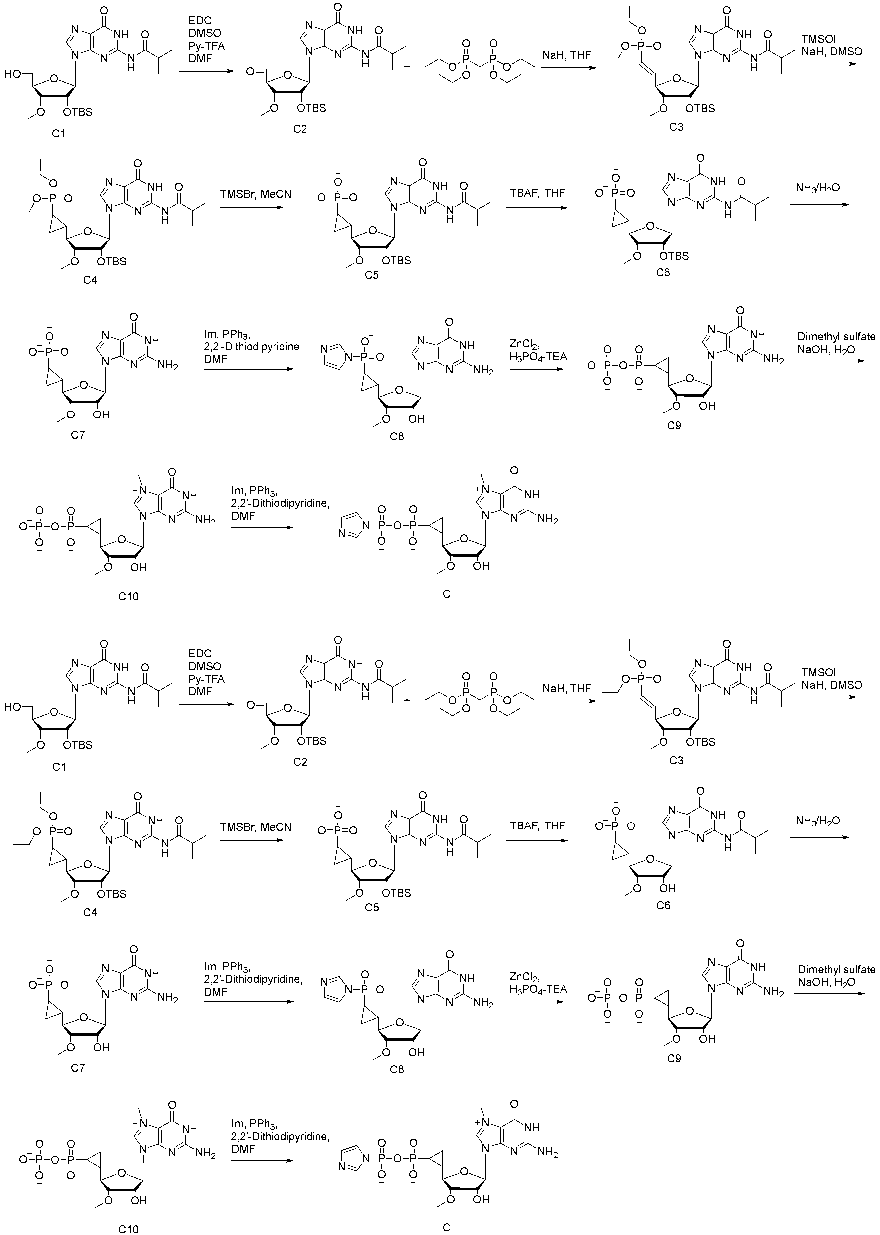

### Example 3: Synthetic Route for Synthesis of Ammonium Salt of Compound 5 Using Intermediates B and D as Starting Materials

With reference to the synthetic method for compound 1 in Example 1, the ammonium salt of compound 5 was obtained using intermediates B and D as starting materials. Reaction route:

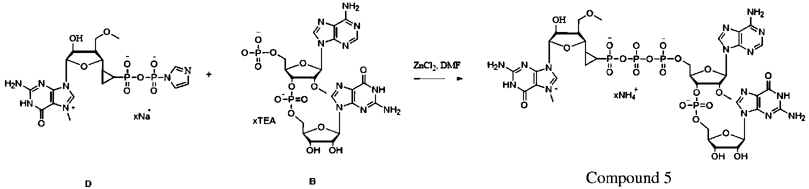

In the route, compound D was obtained by the following steps:
Compound D1 (0.18 mol) and imidazole (0.36 mol) were dissolved in DMF (3.5 L). tert-Butyldiphenylchlorosilane (0.2 mol) was added to the reaction solution under an ice bath. The mixture was stirred at room temperature for 5 h. The reaction solution was poured into ice water, and the mixture was extracted with ethyl acetate (500.0 mL × 2). The organic phase was washed with saturated sodium chloride, then dried, and concentrated under reduced pressure to obtain intermediate D2, which was directly used in the next step without purification.

Acetic anhydride (0.45 mol) and pyridine (0.8 mol) were slowly added to a suspension of CrO₃ (0.45 mol) in dichloromethane (900.0 mL) under an ice-salt bath. The mixture was stirred at room temperature for 15 min, and then intermediate D2 (0.15 mol) was added to the reaction solution. The reaction solution was stirred at room temperature for 1 h and then poured into iced ethyl acetate. The mixture was filtered under vacuum, and the filtrate was concentrated under reduced pressure and then purified by column chromatography to obtain intermediate D3.

Methyltriphenylphosphonium bromide (0.15 mol) was added dropwise to a suspension of NaH (60%, 0.12 mol) in THF (500.0 mL) under an ice bath, and the mixture was stirred at 0 °C for 0.5 h. A solution of intermediate D3 (0.12 mol) in THF (300.0 mL) was slowly added dropwise to the reaction solution. The mixture was stirred at room temperature overnight. A saturated aqueous ammonium chloride solution was added dropwise to the reaction solution under an ice bath to quench the reaction, and the mixture was extracted with ethyl acetate, then concentrated, and purified by column chromatography to obtain intermediate D4.

Intermediate D4 (0.1 mol) was dissolved in anhydrous THF (420.0 mL). Under a nitrogen atmosphere, with the temperature controlled at -5 to 0 °C, the resulting solution was added dropwise to a borane-dimethyl sulfide solution (0.2 mol, 10 M). After the dropwise addition was completed, the mixture was stirred at the same temperature for 6 h. Then, an aqueous sodium hydroxide solution (0.7 mol, 350.0 mL) and hydrogen peroxide (80.0 mL, 30% aqueous solution) were sequentially added dropwise to the reaction solution. The mixture was warmed to room temperature, stirred for 2 h, and then extracted with ethyl acetate (200.0 mL × 2). The organic phase was washed sequentially with water and saturated brine, dried over anhydrous sodium sulfate, concentrated under reduced pressure, and purified by column chromatography to obtain intermediate D5.

A solution of intermediate D5 (85.0 mmol) in THF (200.0 mL) was added dropwise to a suspension of NaH (60%, 95.0 mmol) in THF (170.0 mL) under an ice bath, and the mixture was stirred at 0 °C for 0.5 h. Methyl iodide (100.0 mmol) was added dropwise to the reaction solution. The mixture was stirred at room temperature for 3 h. A saturated aqueous ammonium chloride solution was added dropwise to the reaction solution under an ice bath to quench the reaction, and the mixture was extracted with ethyl acetate, then concentrated, and purified by column chromatography to obtain intermediate D6.

Intermediate D6 (70.0 mmol) was dissolved in anhydrous THF (300.0 mL), and a solution of TBAF in THF (1 M, 0.3 mol) was added. The mixture was then stirred at room temperature for 6 h. The reaction solution was washed with water, and then the aqueous phase was extracted once with ethyl acetate. The organic phases were combined, dried, then concentrated, and purified by column chromatography to obtain intermediate D7.

Intermediate D7 (60.0 mmol) was dissolved in DMF (130.0 mL), and DMSO (0.36 mol) and EDCI (0.18 mol) were added to the reaction solution before pyridine (60.0 mmol) and trifluoroacetic acid (60.0 mmol) were added dropwise. The reaction solution was allowed to react at room temperature for 5 h and then diluted with ethyl acetate. The organic phase was separately washed with a saturated aqueous sodium bicarbonate solution and water, then concentrated, and subjected to column chromatography to obtain intermediate D8.

Tetraethyl methylenediphosphonate (80.0 mmol) was added dropwise to a suspension of NaH (60%, 53.0 mmol) in THF (50.0 mL) under an ice bath, and then the mixture was stirred at 0 °C for 0.5 h. A solution of intermediate D8 (53.0 mmol) in THF (70.0 mL) was slowly added dropwise to the reaction solution. The mixture was stirred at room temperature overnight. A saturated aqueous ammonium chloride solution was added dropwise to the reaction solution under an ice bath to quench the reaction, and the mixture was extracted with ethyl acetate, then concentrated, and subjected to column chromatography to obtain intermediate D9.

Intermediate D9 (44.0 mmol) was dissolved in acetic acid (80.0 mL), and acetic anhydride (132.0 mmol) was added at room temperature. The mixture was cooled to below 10 °C, and concentrated sulfuric acid (6.6 mmol) was added dropwise to the reaction solution. The mixture was stirred at room temperature overnight. The reaction solution was diluted with DCM (150.0 mL), then washed sequentially with water, a saturated aqueous sodium bicarbonate solution, and saturated brine, and dried over anhydrous sodium sulfate. The organic phase was concentrated under reduced pressure to obtain intermediate D10, which was directly used in the next step without purification.

Compound D10 (40.0 mmol) and compound D11 (40.0 mmol) were weighed and suspended in DCE (300.0 mL), and BSA (0.1 mol) was added to the reaction solution. The reaction mixture was heated to 70 °C, stirred for 1 h, and then cooled to 0 °C. TMSOTf (0.12 mol) was added dropwise to the reaction solution. After the dropwise addition was completed, the mixture was heated to 70 °C and stirred for 3 h. The reaction solution was cooled to room temperature and then washed with a saturated aqueous sodium bicarbonate solution. The organic phase was concentrated under reduced pressure and then purified by column chromatography to obtain intermediate D12.

With reference to the synthetic method for intermediate C4, intermediate D13 was obtained.

With reference to the synthetic method for intermediate C5, intermediate D14 was obtained.

With reference to the synthetic method for intermediate C7, intermediate D15 was obtained.

With reference to the synthetic method for intermediate C8, intermediate D16 was obtained.

With reference to the synthetic method for intermediate C9, intermediate D17 was obtained.

With reference to the synthetic method for intermediate C10, intermediate D18 was obtained.

With reference to the synthetic method for intermediate C, intermediate D was obtained.

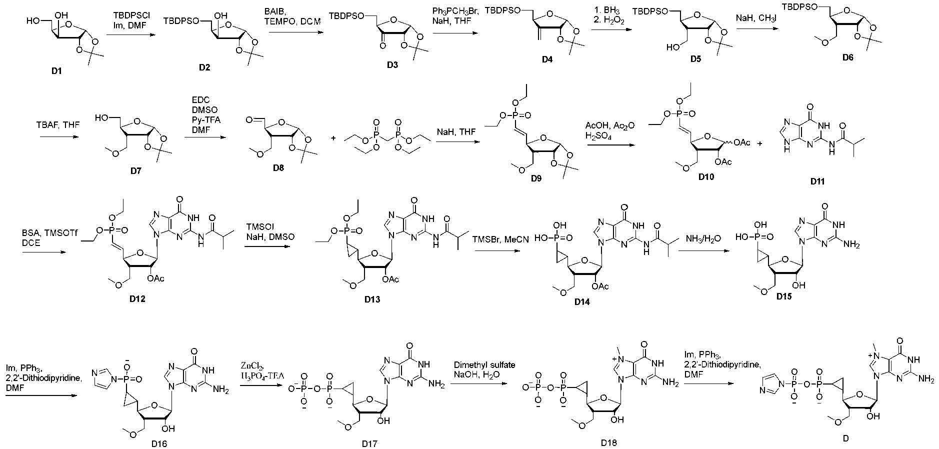

### Example 4: Synthetic Route for Synthesis of Ammonium Salt of Compound 9 Using Intermediates B and E as Starting Materials

With reference to the synthetic method for compound 1 in Example 1, the ammonium salt of compound 9 was obtained using intermediates B and E as starting materials. The reaction route is shown as follows:

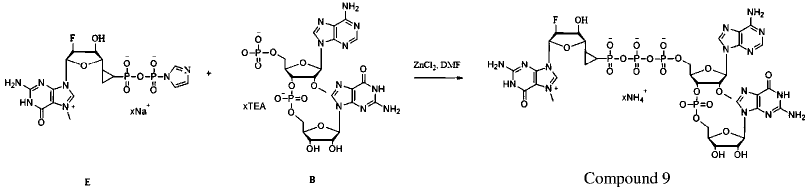

In the route, compound E was obtained by the following steps: With reference to the synthetic methods for intermediates C2-C in Example 2, intermediates E2-E were obtained. Reaction route:

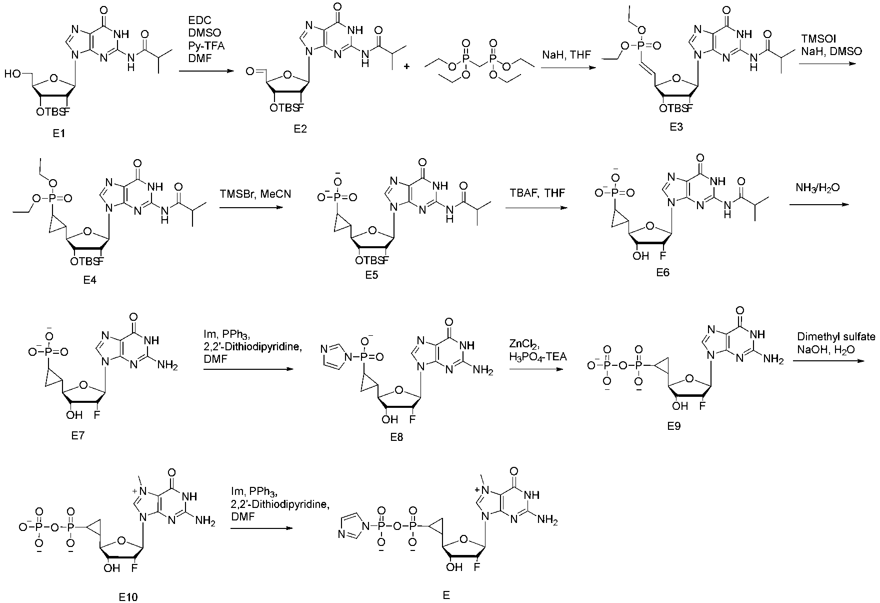

### Example 5: Synthetic Route for Synthesis of Ammonium Salt of Compound 10 Using Intermediates B and F as Starting Materials

With reference to the synthetic method for compound 1 in Example 1, the ammonium salt of compound 10 was obtained using intermediates B and F as starting materials. Reaction route:

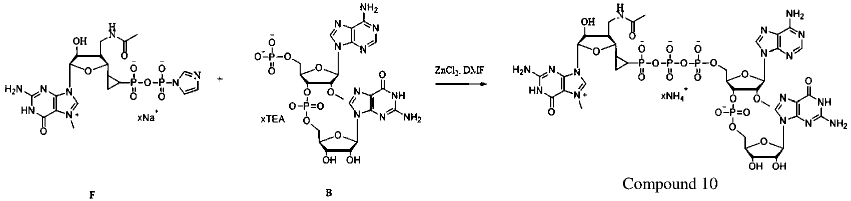

In the route, compound F was obtained by the following steps:
Intermediate D5 (0.33 mol), triphenylphosphine (0.66 mol), and DIAD (0.66 mol) were dissolved in THF (1.5 L). DPPA (0.66 mol) was added dropwise to the reaction solution. The mixture was stirred at room temperature overnight, concentrated under reduced pressure to remove the solvent, and purified by column chromatography to obtain intermediate F1.

With reference to the synthetic method for intermediate D7 in Example 3, intermediate F2 was obtained.

With reference to the synthetic method for intermediate D8 in Example 3, intermediate F3 was obtained.

With reference to the synthetic method for intermediate D9 in Example 3, intermediate F4 was obtained.

With reference to the synthetic method for intermediate D10 in Example 3, intermediate F5 was obtained.

With reference to the synthetic method for intermediate D12 in Example 3, intermediate F6 was obtained.

With reference to the synthetic method for intermediate D13 in Example 3, intermediate F7 was obtained.

With reference to the synthetic method for intermediate D15 in Example 3, intermediate F8 was obtained.

Intermediate F8 (125.0 mmol) was dissolved in THF (1.0 L). Water (100.0 mL) and triphenylphosphine (187.5 mmol) were then added, and the mixture was heated to 50 °C, stirred overnight, and concentrated to remove the solvent. The crude product was purified by column chromatography to obtain intermediate F9.

Intermediate F9 (108.0 mmol) was dissolved in DCM (940.0 mL), and TEA (143.0 mmol) was added. Benzoyl chloride (130.0 mmol) was then added dropwise to the reaction solution under an ice bath. The mixture was warmed to room temperature and then stirred for 2 h. The reaction solution was washed sequentially with water and saturated brine, and dried over anhydrous sodium sulfate. The organic phase was concentrated under reduced pressure and purified by column chromatography to obtain intermediate F10.

With reference to the synthetic method for intermediate D14 in Example 3, intermediate F11 was obtained.

With reference to the synthetic method for intermediate D16 in Example 3, intermediate F12 was obtained.

With reference to the synthetic method for intermediate D17 in Example 3, intermediate F13 was obtained.

With reference to the synthetic method for intermediate D18 in Example 3, intermediate F14 was obtained.

With reference to the synthetic method for intermediate D in Example 3, intermediate F was obtained.

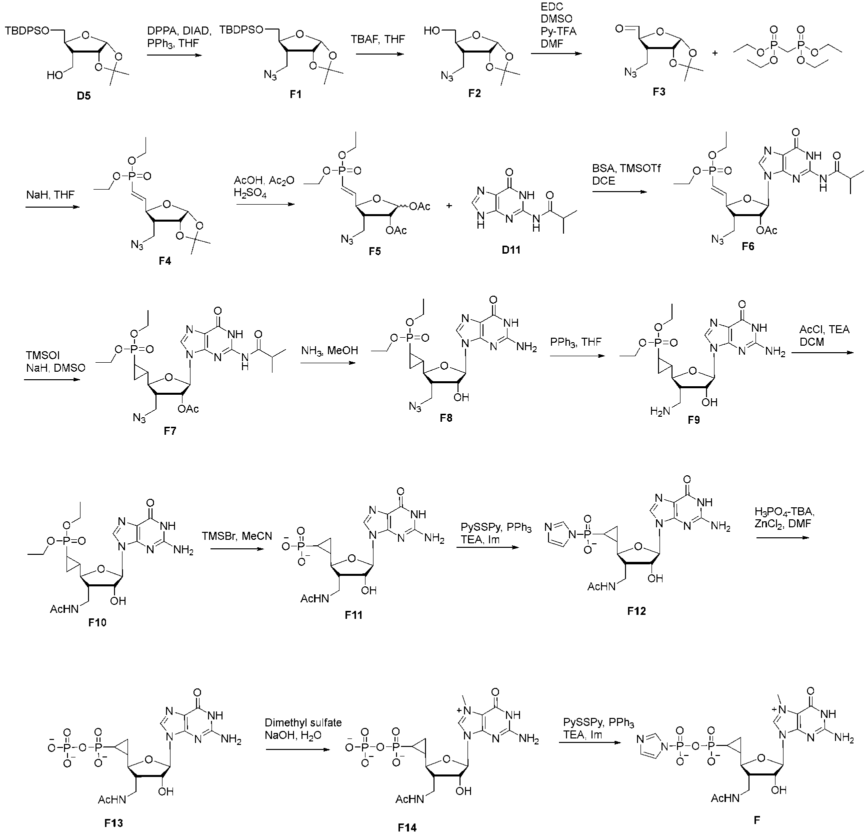

### Example 6: Synthetic Route for Synthesis of Ammonium Salt of Compound 14 Using Intermediates A and G as Starting Materials

With reference to the synthetic method for compound 1 in Example 1, the ammonium salt of compound 14 was obtained using intermediates A and G as starting materials. Reaction route:

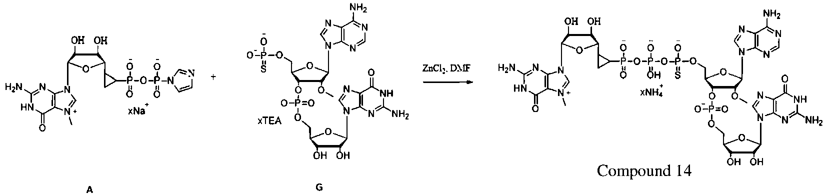

In the route, compound G was obtained by the following steps:
Intermediate B1 (0.18 mol) and bis(2-cyanoethyl)-N,N-diisopropylphosphoramidite (0.25 mol) were dissolved in DCM (1.7 L). Under a nitrogen atmosphere, tetrazole (0.39 mol) was added. The mixture was stirred at room temperature for 2 h. After the reaction was completed, DDTT (0.51 mol) was added to the reaction solution, and the mixture was allowed to react at room temperature for another 1 h. The reaction solution was separately washed with a 10% aqueous sodium sulfite solution, a 10% aqueous sodium bicarbonate solution, and saturated brine. The organic phase was concentrated under reduced pressure, and then the residue was dissolved in methanol (0.5 L) and aqueous ammonia (1.0 L). The resulting solution was stirred at room temperature overnight, concentrated to remove the solvent, then diluted with water, loaded onto DEAE Sephadex, and eluted with a linear gradient of a 0-1.0 M TEAB eluent. The eluate was concentrated to obtain intermediate G. Reaction route:

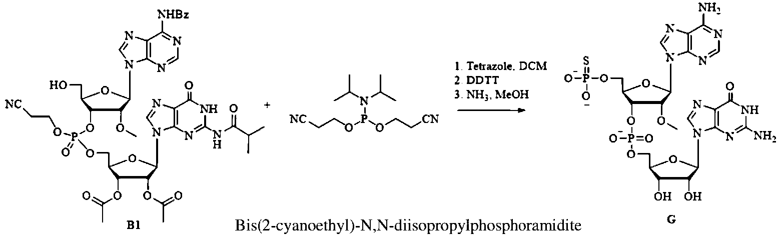

### Example 7: Synthetic Route for Synthesis of Ammonium Salt of Compound 21 Using Intermediates H and I as Starting Materials

With reference to the synthetic method for compound 1 in Example 1, the ammonium salt of compound 11 was obtained using intermediates H and I as starting materials. Reaction route:

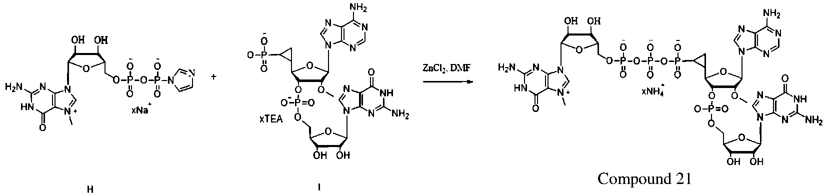

In the route, compound H was obtained by the following steps: With reference to the synthetic methods for intermediates F14 and F in Example 5, intermediates Y1-Y were obtained, respectively. Reaction route:

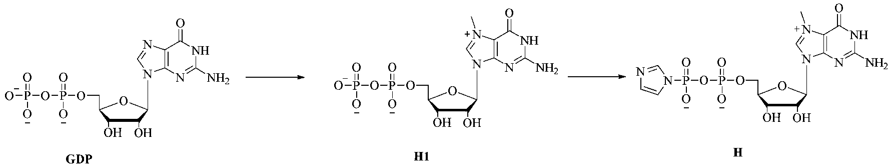

Compound I was obtained by the following steps:
Intermediate B1 (0.3 mol) was dissolved in DMF (1.2 L), and DMSO (1.8 mol) and EDCI (0.9 mol) were added to the reaction solution before pyridine (0.3 mol) and trifluoroacetic acid (0.3 mol) were added dropwise. The reaction solution was allowed to react at room temperature for 5 h and then diluted with ethyl acetate. The organic phase was separately washed with a saturated aqueous sodium bicarbonate solution and water, then concentrated, and subjected to column chromatography to obtain intermediate I1.

Tetraethyl methylenediphosphonate (0.24 mol) was added dropwise to a suspension of NaH (60%, 0.16 mol) in THF (500.0 mL) under an ice bath, and then the mixture was stirred at 0 °C for 0.5 h. A solution of intermediate I1 (0.16 mol) in THF (400.0 mL) was slowly added dropwise to the reaction solution. The mixture was stirred at room temperature overnight. A saturated aqueous ammonium chloride solution was added dropwise to the reaction solution under an ice bath to quench the reaction, and the mixture was extracted with ethyl acetate, then concentrated, and subjected to column chromatography to obtain intermediate I2.

With reference to the synthetic method for intermediate F7 in Example 5, intermediate I3 was obtained.

With reference to the synthetic method for intermediate F11 in Example 5, intermediate I4 was obtained.

With reference to the synthetic method for intermediate G in Example 6, intermediate I was obtained.

### Reaction route:

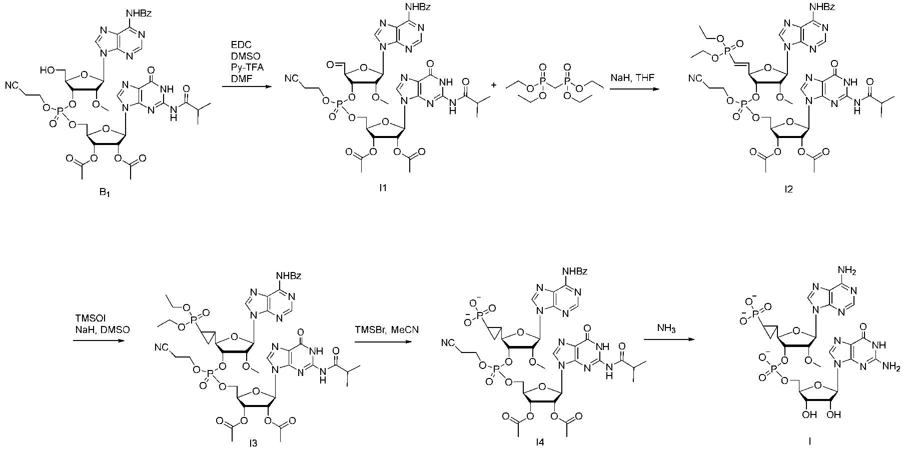

### Example 8: Synthetic Route for Synthesis of Ammonium Salt of Compound 22 Using Intermediates H and J as Starting Materials

With reference to the synthetic method for compound 1 in Example 1, the ammonium salt of compound 22 was obtained using intermediates H and J as starting materials. Reaction route:

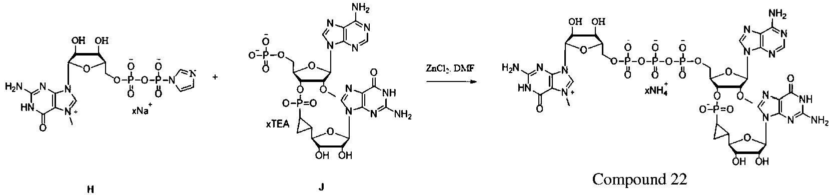

In the route, compound J was obtained by the following steps:

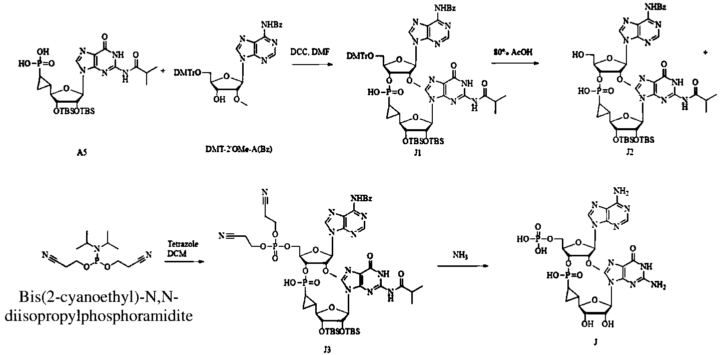

Intermediate A5 (0.21 mol) and DMT-2'OMe-A(Bz) (0.25 mol) were dissolved in DMF (1.5 L), and DCC (0.5 mol) was added to the reaction solution. The mixture was allowed to react at room temperature overnight. After the reaction was completed, water (4.5 L) was added to quench the reaction, and the mixture was extracted with ethyl acetate (500.0 mL × 3). The organic phases were combined, then concentrated under reduced pressure, and purified by column chromatography to obtain intermediate J1.

Intermediate J1 (0.11 mol) was dissolved in an 80% aqueous acetic acid solution (1.6 L), and the resulting solution was stirred at room temperature overnight. After the reaction was completed, the reaction solution was concentrated under reduced pressure to remove the solvent and then purified by column chromatography to obtain intermediate J2.

With reference to the synthetic method for intermediate B in Example 1, intermediate J was obtained.

### Example 9: Synthetic Route for Synthesis of Ammonium Salt of Compound 31 Using Intermediates B and K as Starting Materials

With reference to the synthetic method for compound 1 in Example 1, the ammonium salt of compound 31 was obtained using intermediates B and K as starting materials. Reaction route:

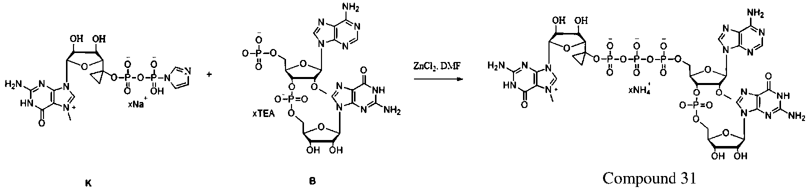

In the route, compound K was obtained by the following steps:
Intermediate A2 (0.19 mol) was dissolved in acetonitrile (1.1 L), and an aqueous sodium dihydrogen phosphate solution (0.2 M, 38.2 mmol) and hydrogen peroxide (30 wt.%, 0.21 mol) were added to the reaction solution. The reaction solution was cooled to 0 °C, and a sodium chlorite solution (0.75 M, 0.29 mol) was added dropwise thereto. After the dropwise addition was completed, the mixture was warmed to room temperature and stirred for 1 h. An aqueous sodium sulfite solution was added to the reaction solution to quench the reaction. The reaction solution was concentrated to obtain a crude product of intermediate K1.

The crude product of intermediate K1 was dissolved in DMF (1.0 L). The reaction solution was cooled to 0 °C, and NaHCO₃ (1.35 mol) and methyl iodide (0.34 mol) were separately added thereto. The mixture was stirred at room temperature overnight. The reaction solution was diluted with water and then extracted with ethyl acetate (500.0 mL × 2). The organic phases were combined, then concentrated under reduced pressure, and purified by column chromatography to obtain intermediate K2.

Intermediate K2 (0.1 mol) was dissolved in anhydrous THF (610.0 mL). The resulting solution was cooled to 0 °C. Under a nitrogen atmosphere, tetraisopropyl titanate (0.1 mol) and ethylmagnesium bromide (2.0 M in THF, 0.5 mol) were added. The mixture was warmed to room temperature and stirred for 6 h. A saturated aqueous ammonium chloride solution was added to the reaction solution, followed by liquid separation. The aqueous phase was filtered under vacuum and then extracted with ethyl acetate. The organic phases were combined and then concentrated under reduced pressure. The crude product was purified by column chromatography to obtain intermediate K3.

With reference to the synthetic method for intermediate E6 in Example 4, intermediate K4 was obtained.

With reference to the synthetic method for intermediate E7 in Example 4, intermediate K5 was obtained.

Intermediate A3 (0.55 mol) was dissolved in trimethyl phosphate (200.0 mL). The resulting solution was cooled to -0 °C, and then phosphorus oxychloride (0.1 mol) was added dropwise to the reaction solution. The mixture was stirred at -0 °C for 3 h, and then a TEAB solution was added dropwise to the reaction solution to quench the reaction. The mixture was then purified by reverse-phase preparative chromatography to obtain intermediate K5.

With reference to the synthetic method for intermediate E8 in Example 4, intermediate K7 was obtained.

With reference to the synthetic method for intermediate E9 in Example 4, intermediate K8 was obtained.

With reference to the synthetic method for intermediate E10 in Example 4, intermediate K9 was obtained.

With reference to the synthetic method for intermediate E in Example 4, intermediate K was obtained.

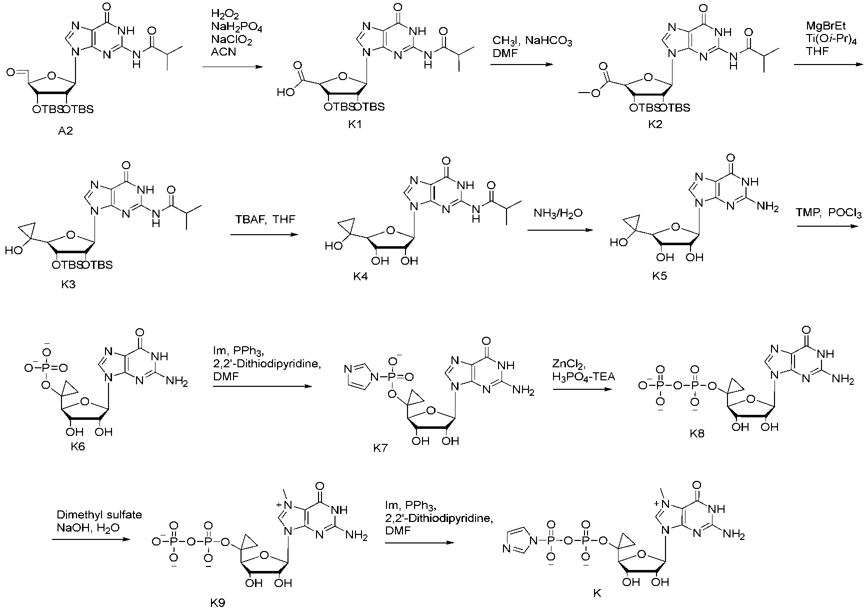

### Example 10: Synthetic Route for Synthesis of Ammonium Salt of Compound 32 Using Intermediates B and L as Starting Materials

With reference to the synthetic method for compound 1 in Example 1, the ammonium salt of compound 32 was obtained using intermediates B and L as starting materials. Reaction route:

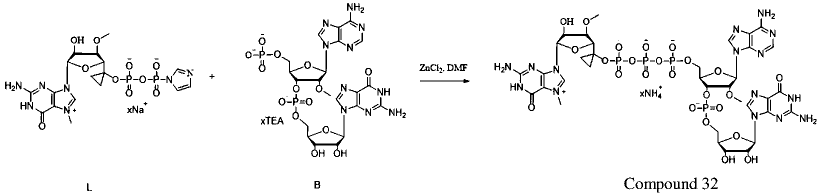

In the route, compound L was obtained by the following steps: With reference to the synthetic methods for intermediates K1-K in Example 9, intermediates L1-L were obtained.

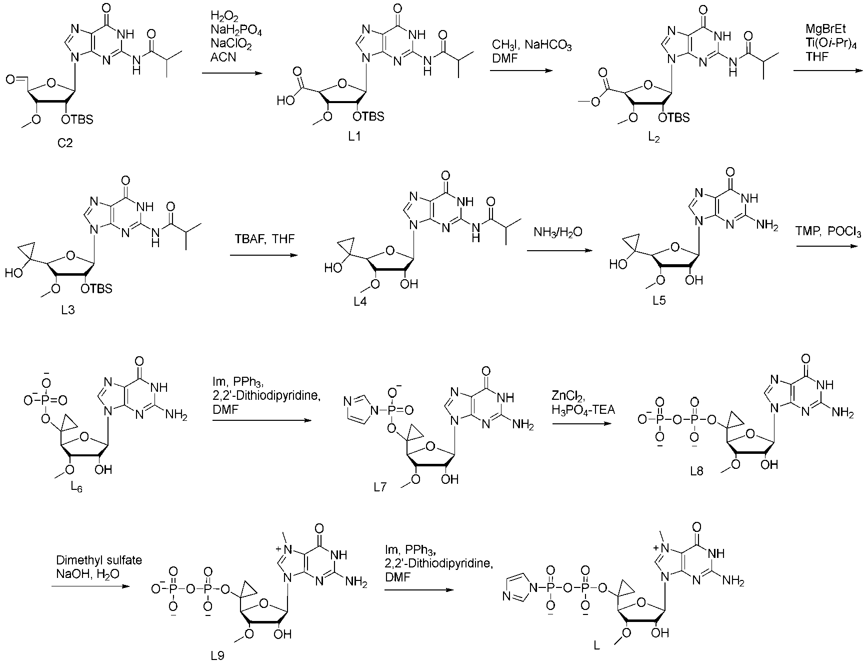

### Example 11: Synthetic Route for Synthesis of Ammonium Salt of Compound 37 Using Intermediates K and M as Starting Materials

With reference to the synthetic method for compound 1 in Example 1, the ammonium salt of compound 37 was obtained using intermediates K and M as starting materials. Reaction route:

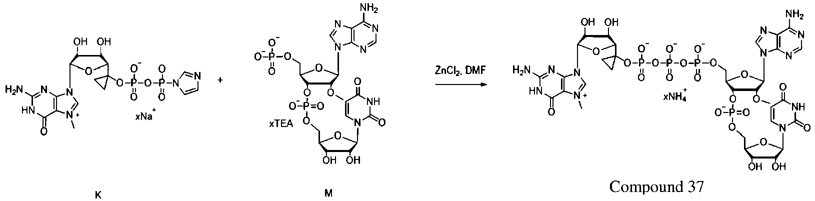

In the route, compound M was obtained by the following steps: With reference to the synthetic method for intermediate B in Example 1, intermediate M was obtained.

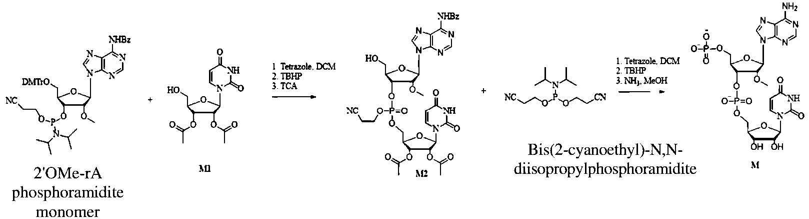

### Example 12: Synthetic Route for Synthesis of Ammonium Salt of Compound 38 Using Intermediates A and M as Starting Materials

With reference to the synthetic method for compound 1 in Example 1, the ammonium salt of compound 38 was obtained using intermediates A and M as starting materials. Reaction route:

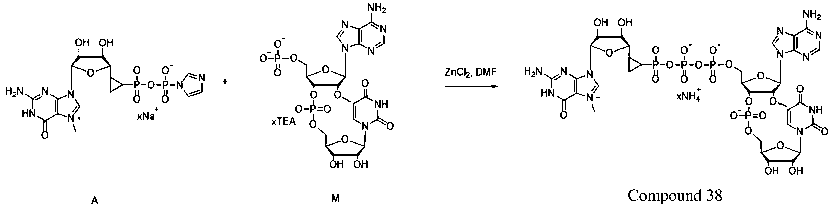

### Comparative Example 1: Synthetic Route for Synthesis of Ammonium Salt of Compound 43 Using Intermediates H and B as Starting Materials

With reference to the synthetic method for compound 1 in Example 1, the ammonium salt of compound 43 was obtained using intermediates H and B as starting materials. Reaction route:

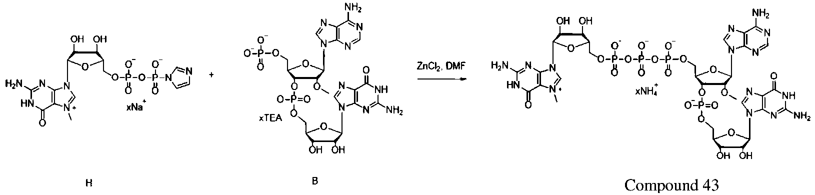

### Biological Activity Assay Section:

### Test Example 1: Determination of mRNA In Vitro Transcription Yield and Capping Efficiency

mRNA IVT reactions were performed using the cap analogs from the examples. Firstly, the required volumes of materials for the system were calculated, and then the samples were added (the IVT reaction system is shown in Table 1). Sterile enzyme-free water was added to the system, and then 10× buffer, NTPs, and the cap analog were sequentially added. The mixture was uniformly mixed and then gently centrifuged, followed by the addition of the nuclease inhibitor, the inorganic pyrophosphatase, the T7 RNA polymerase, and the linearized DNA template. The resulting mixture was thoroughly mixed, then gently centrifuged, and incubated at 37 °C. After 2 h, DNase I (1 U) was added, and the mixture was incubated at 37 °C for another 30 min to remove the DNA template. RNA purification was then performed using a magnetic bead purification method. The purified mRNA was dissolved in sterile enzyme-free water, and then quantitative detection was performed using Nanodrop One.

**Table 1. IVT reaction system**

| IVT reaction system | Amount |
|---|---|
| T7 RNA polymerase | 50 U |
| 10Xbuffer | 2 µl |
| 100mMATP | 1 µl |
| 100mMGTP | 1 µl |
| 100mMCTP | 1 µl |
| 100mMN1-Me-pUTP | 1 µl |
| 100 mM cap analog | 1 µl |
| Inorganic pyrophosphatase | 0.05 U |
| Nuclease inhibitor | 20 U |
| Sterile enzyme-free water | Making up to 20 µL |
| DNA template | 1 µg |

Liquid chromatography-mass spectrometry (LC-MS) was used to detect the IVT capping rates of mRNAs with different starting cap analogs. Firstly, a labeled (usually biotin-labeled) DNA probe matched with the starting base of the transcribed mRNA product was required to be designed, and streptavidin-labeled magnetic beads were washed, and then incubated with the synthesized DNA probe, mRNA, and 10× RNase H reaction buffer with slow mixing at room temperature for 30 min. Subsequently, 20 µL of RNase H (5 U/µL) was added, and the mixture was incubated at 37 °C for 3 h, with uniform mixing every half hour. After the incubation was completed, the magnetic beads were washed, and then 100 µL of 75% methanol heated to 80 °C was added to the washed magnetic beads. The mixture was heated to 80 °C on a heating plate, held for 3 min, and then placed on a magnetic rack. The supernatant was pipetted and dried at room temperature for 45 min to 10 µL by using an evaporation centrifuge. The sample was then resuspended in 50 µL of 100 µM EDTA/1% MeOH for LC-MS analysis to determine the capping of RNA in the transcription reaction. Since capped and uncapped bases have a distinct difference in molecular weight, the capping rates of mRNA transcription processes initiated with different cap analogs can be determined by utilizing this difference in molecular mass.

The specific results are shown in Table 2.

**Table 2. mRNA yield and capping efficiency**

| Example | Compound No. | Yield (µg) in 20 µL system | Capping rate (%) |
|---|---|---|---|
| Example 1 | Compound 1 | 114 | 99.1 |
| Example 2 | Compound 2 | 117 | 98.4 |
| Example 3 | Compound 5 | 110 | 96.3 |
| Example 4 | Compound 9 | 98 | 94.5 |
| Example 5 | Compound 10 | 103 | 96.3 |
| Example 6 | Compound 14 | 102 | 98.0 |
| Example 7 | Compound 21 | 99 | 97.1 |
| Example 8 | Compound 22 | 115 | 96.0 |
| Example 9 | Compound 31 | 112 | 98.5 |
| Example 10 | Compound 32 | 116 | 98.9 |
| Comparative Example 1 | Compound 43 | 105 | 96.1 |

From the experimental results, it can be seen that compared to Comparative Example 1, the use of the cap analogs of the present application for mRNA IVT reactions significantly improved both the mRNA *in vitro* transcription yield and capping efficiency.

### Test Example 2: mRNA Translation Efficiency

Test method: *In vitro* transcription was initiated with the cap analogs from the examples and Comparative Example 1 by using an eGFP coding sequence as a DNA template. The different mRNA products obtained were then transfected into 293T cells. The 293T cells were plated (onto a 24-well plate) at (0.5-1) × 10⁵ cells/well. During the transfection, in general, the cell density was preferably 60%-80%; each well was transfected with 2 µg of mRNA, and the Lipofectamine MessengerMAX Transfection Reagent (Invitrogen) was selected as the transfection reagent and used according to its instructions for use. After transfection, the cells were placed in an incubator at 37 °C with CO₂. After transfection for 4-6 h, the medium was replaced with a fresh complete medium. After incubation in the incubator at 37 °C with CO₂ for 24 h, the GFP fluorescence intensity in the cells was observed under a fluorescence microscope, and the fluorescence intensity ratio of each example relative to Comparative Example 1 was calculated based on the fluorescence intensity.

**Table 3. mRNA translation efficiency**

| Example | Compound No. | Relative fluorescence intensity (relative to Comparative Example 1) |
|---|---|---|
| Example 1 | Compound 1 | 2.26 |
| Example 2 | Compound 2 | 2.58 |
| Example 3 | Compound 5 | 1.98 |
| Example 4 | Compound 9 | 2.12 |
| Example 5 | Compound 10 | 2.16 |
| Example 6 | Compound 14 | 1.44 |
| Example 7 | Compound 21 | 1.37 |
| Example 8 | Compound 22 | 1.24 |
| Example 9 | Compound 31 | 1.84 |
| Example 10 | Compound 32 | 1.94 |
| Comparative Example 1 | Compound 43 | 1.0 |

From the experimental results, it can be seen that when *in vitro* transcription was initiated with the cap analogs of the present application, the mRNA translation efficiency was higher than that of Comparative Example 1 after transfection and 24-hour incubation of the cells. The above results indicate that the cap analogs of the present application exhibited higher translation efficiency at the cellular level.

### Test Example 3: mRNA Translation Efficiency in Mice

### Experimental procedures:

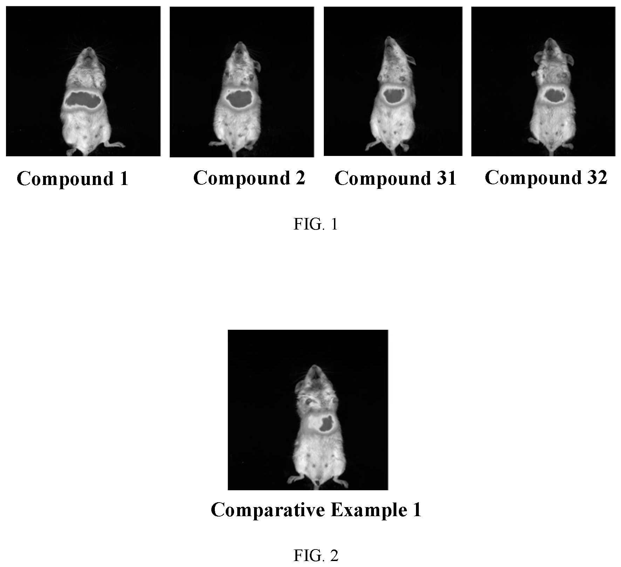

The tail of each mouse was gently wiped with an alcohol cotton ball, and 1 µg of an LNP-mRNA drug was injected via the tail vein using an insulin syringe. Twenty-four hours after the tail vein administration, the mouse was intraperitoneally injected with 10% chloral hydrate (anesthetized according to the mouse body weight, 50-60 µL/20 g). After the mouse was anesthetized, 150 µL of a prepared luminescent substrate (luminescent substrate concentration: 15 mg/mL, Cat. No. 2109GR001, brand: biofroxx) was intraperitoneally injected. Five minutes after the injection of the luminescent substrate into the mouse, the mouse was placed in a small animal *in vivo* imaging system. The corresponding luciferase channel was selected to capture an *in vivo* image of the mouse, and the image was saved. This procedure was repeated at other time points, and the exposure parameters should be kept consistent for comparison. The imaging results reflecting mRNA translation efficiency in mice are shown in FIG. 1.

From FIG. 1, it can be seen that after the tail vein administration, compounds 1, 2, 31, and 32, and Comparative Example 1 were mainly distributed in the liver. Twenty-four hours after the administration, the modified mRNAs of compounds 1, 2, 31, and 32 showed higher protein expression levels in mice compared to Comparative Example 1.

Although the embodiments of the present disclosure have been shown and described, it will be understood by those of ordinary skill in the art that various changes, modifications, substitutions, and alterations can be made to these embodiments without departing from the principle and spirit of the present disclosure, and the scope of the present disclosure is defined by the appended claims and equivalents thereof.

## Claims

1. A capping compound, being a compound having a structure represented by formula (I), or a stereoisomer, a pharmaceutically acceptable salt, or a solvate thereof, 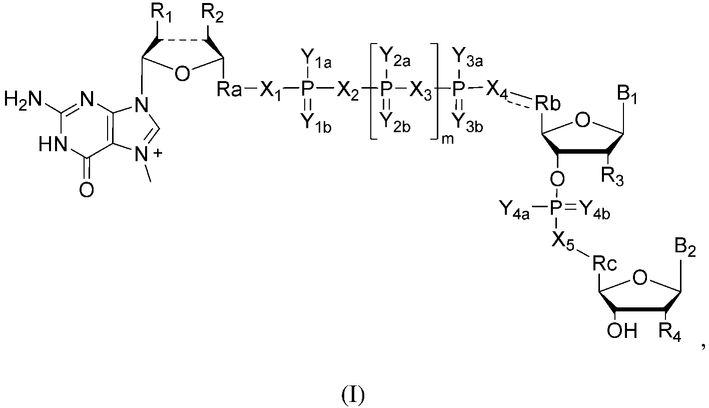
wherein - - - represents a single bond or none;
-̅ -̅ -̅ represents a single bond or a double bond;
R₁ and R₂ are each independently H, OH, halogen, alkyl, alkoxy, alkenyl, alkynyl, cycloalkyl, cycloalkenyl, heterocyclyl, heteroaryl, alkylthio, or alkoxy forming a bridged ring with the carbon at 4', wherein the alkyl, alkoxy, alkenyl, alkynyl, cycloalkyl, cycloalkenyl, heterocyclyl, heteroaryl, and alkylthio are unsubstituted or substituted with one or more R₅, and R₅ is any one of alkyl, alkoxy, and substituted or unsubstituted acetamido;
R₃ is H, OH, halogen, alkoxy, or alkoxy forming a bridged ring with the carbon at 4', wherein the alkoxy is unsubstituted or substituted with one or more R₆, and R₆ is any one of alkyl and alkoxy;
R₄ is H, OH, alkyl, or alkoxy, wherein the alkyl and alkoxy are unsubstituted or substituted with one or more R₇, and R₇ is any one of alkyl and alkoxy;
Ra, Rb, and Rc are each independently 3- to 6-membered cycloalkyl, 3- to 6-membered heterocyclyl, CH₂, CH, or O, wherein Ra and X₁ are not simultaneously O, Rb and X₄ are not simultaneously O, and Rc and X₅ are not simultaneously O; and at least one of Ra, Rb, and Rc is 3- to 6-membered cycloalkyl or 3- to 6-membered heterocyclyl;
X₁, X₂, X₃, X₄, and X₅ are each independently selected from O, S, CH₂, CH, and NH, or absent;
Y₁ₐ, Y₂ₐ, Y₃ₐ, and Y₄ₐ are each independently OH, SH, or BH₃;
Y_{1b}, Y_{2b}, Y_{3b}, and Y_{4b} are each independently O or S;
m is 0, 1, 2, or 3;
B₁ and B₂ are each independently a natural or modified nucleobase.

2. The capping compound according to claim 1, wherein any one or two of Ra, Rb, and Rc are 3- to 6-membered cycloalkyl or 3- to 6-membered heterocyclyl.

3. The capping compound according to claim 2, wherein
any one or two of Ra, Rb, and Rc are three-membered cycloalkyl or four-membered cycloalkyl having a structure selected from: 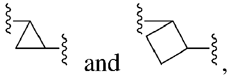 and X₁, X₄, or X₅ adjacent thereto is absent.

4. The capping compound according to claim 3, being a compound having a structure represented by formula (II), or a stereoisomer, a pharmaceutically acceptable salt, or a solvate thereof, 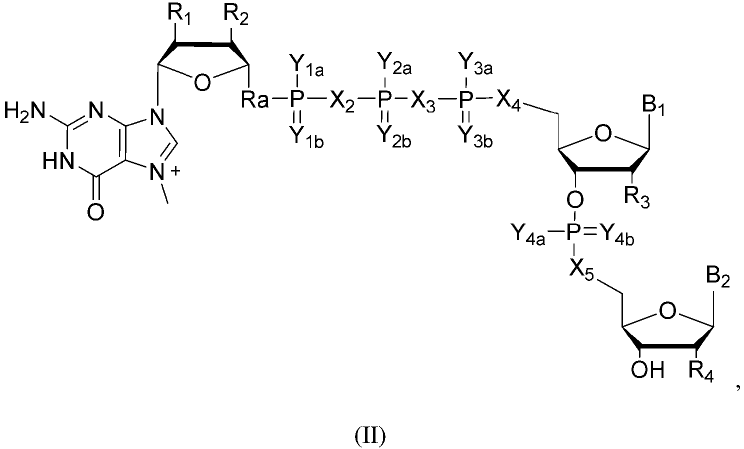 wherein Ra is selected from

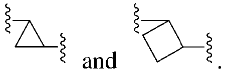

5. The capping compound according to claim 1, wherein R₃ is H, OH, C₁₋₆ alkoxy, or LNA, wherein the C₁₋₆ alkoxy is unsubstituted or substituted with one or more R₆, and R₆ is C₁₋₆ alkyl or C₁₋₆ alkoxy.

6. The capping compound according to claim 5, wherein R₃ is H, OH, or C₁₋₄ alkoxy, and R₄ is H, OH, or C₁₋₄ alkoxy.

7. The capping compound according to claim 1, wherein R₁ and R₂ are each independently H, OH, halogen, C₁₋₆ alkyl, C₁₋₆ alkoxy, or LNA, wherein the C₁₋₆ alkyl and C₁₋₆ alkoxy are unsubstituted or substituted with one or more R₅, and R₅ is any one of C₁₋₆ alkyl, C₁₋₆ alkoxy, and substituted or unsubstituted acetamido.

8. The capping compound according to claim 7, wherein R₁ and R₂ are each independently OH or C₁₋₄ alkoxy, wherein the C₁.₄ alkoxy is unsubstituted or substituted with one or two R₅, and R₅ is C₁.₄ alkyl or C₁.₄ alkoxy.

9. The capping compound according to claim 8, being a compound having a structure represented by formula (III), or a stereoisomer, a pharmaceutically acceptable salt, or a solvate thereof,

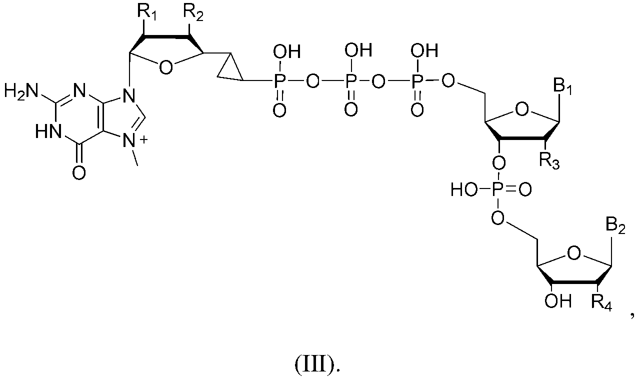

10. The capping compound according to claim 1, wherein any one or two of Ra, Rb, and Rc are three-membered cycloalkyl or four-membered cycloalkyl having a structure selected from: 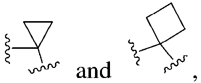 and X₁, X₂, or X₅ adjacent thereto is O.

11. The capping compound according to claim 9, being a compound having a structure represented by formula (IV), or a stereoisomer, a pharmaceutically acceptable salt, or a solvate thereof, 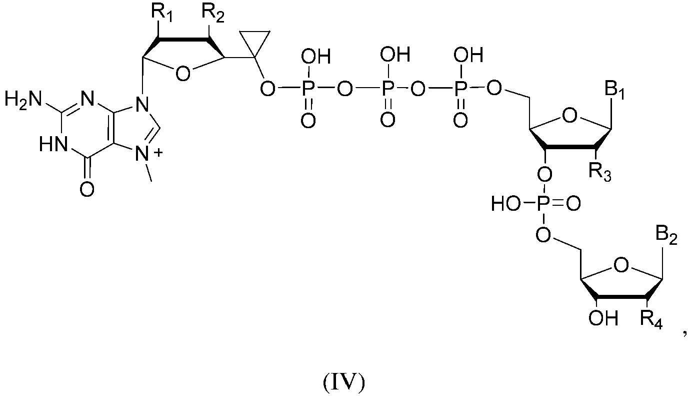
wherein R₁ and R₂ are each independently OH or C₁₋₄ alkoxy, wherein the C₁₋₄ alkoxy is unsubstituted or substituted with one or more R₅, and R₅ is C₁₋₄ alkyl or C₁₋₄ alkoxy;
R₃ is C₁₋₆ alkoxy, wherein the C₁₋₆ alkoxy is unsubstituted or substituted with one or more R₆, and R₆ is C₁₋₆ alkyl or C₁₋₆ alkoxy;
R₄ is H, OH, or C₁₋₄ alkoxy.

12. The capping compound according to any one of claims 1-11, wherein the capping compound is selected from a compound represented by any one of the following, or a stereoisomer, a pharmaceutically acceptable salt, or a solvate thereof,

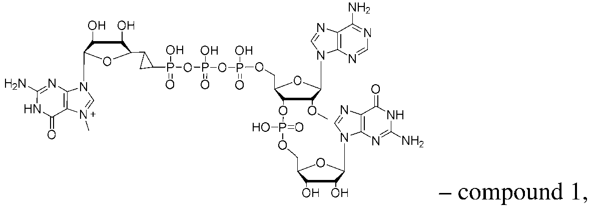

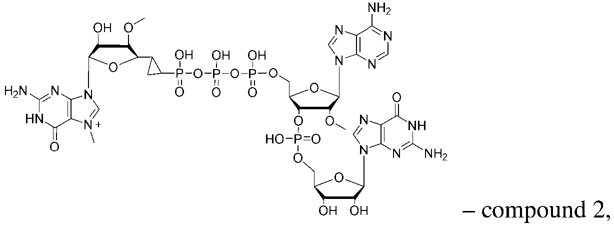

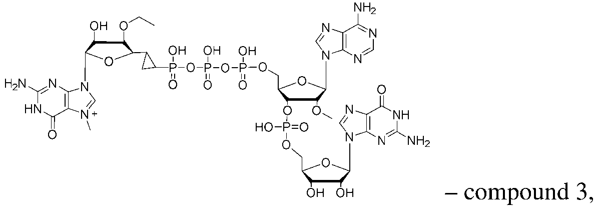

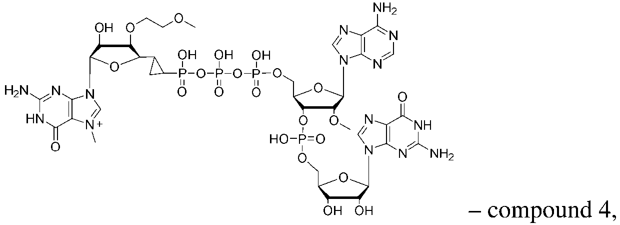

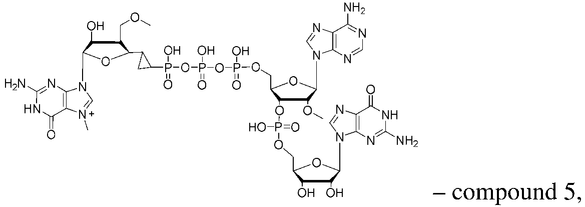

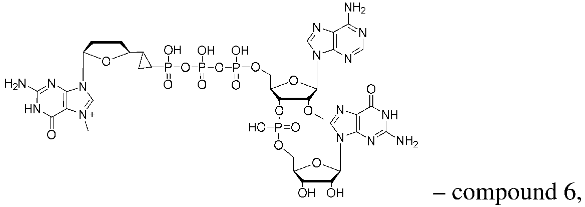

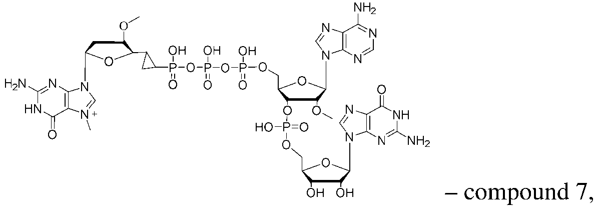

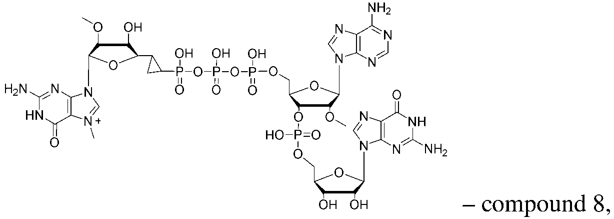

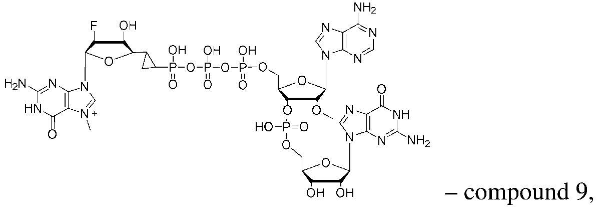

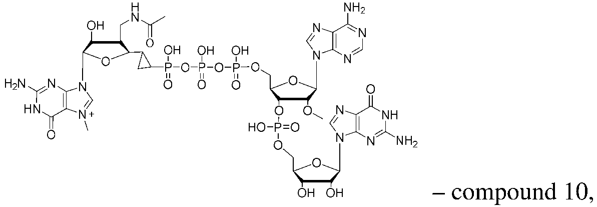

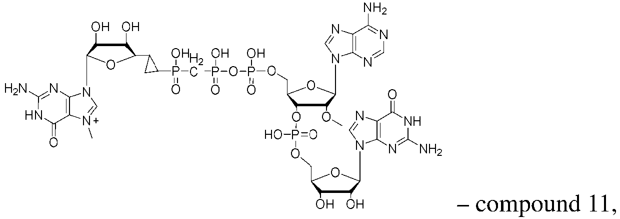

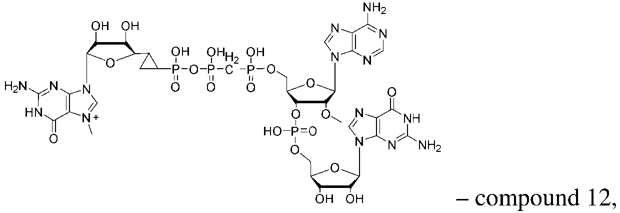

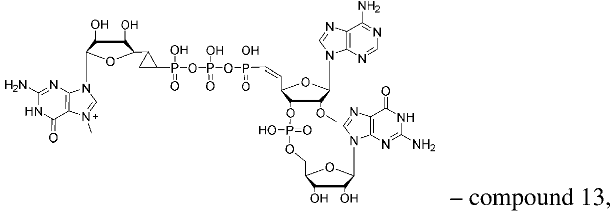

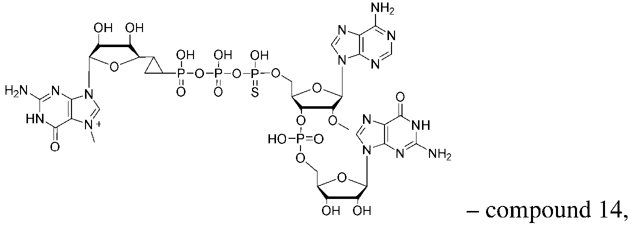

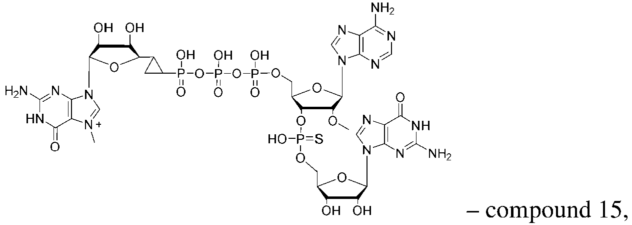

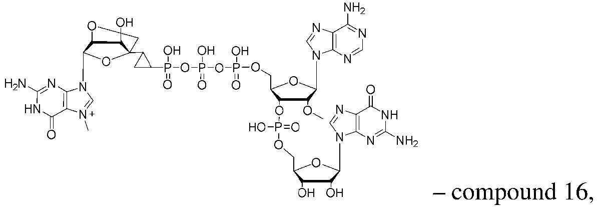

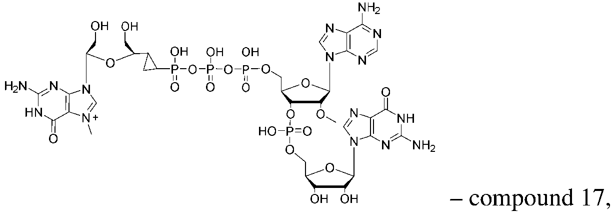

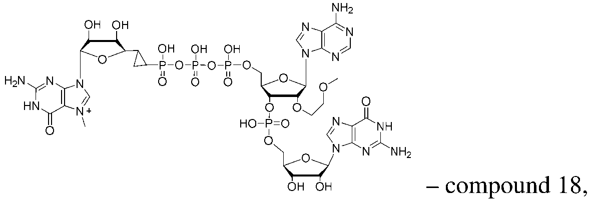

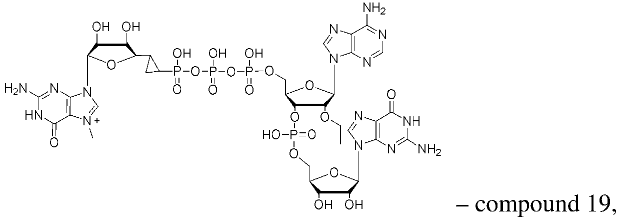

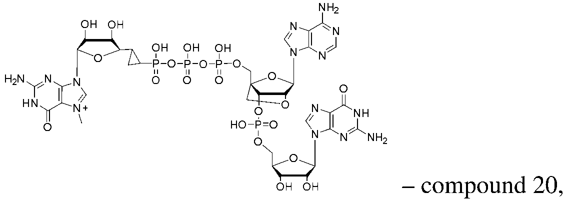

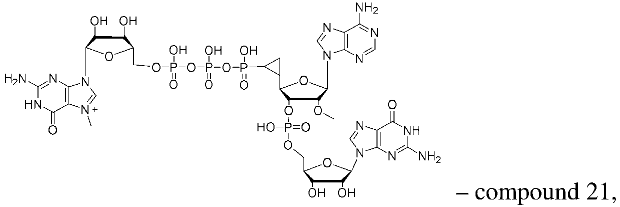

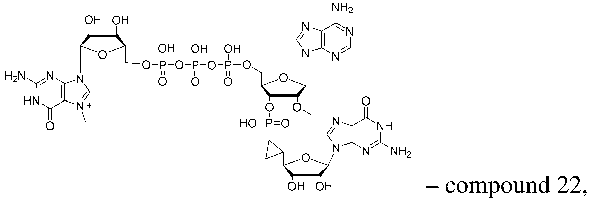

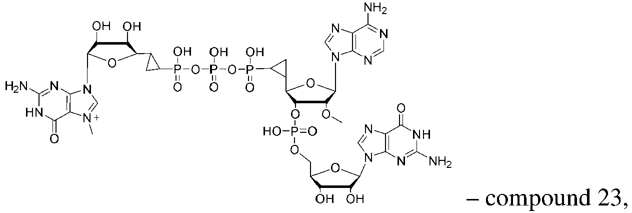

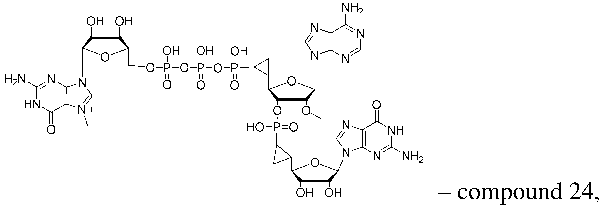

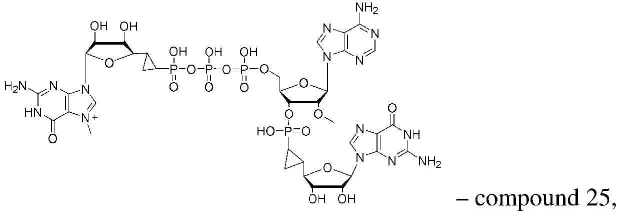

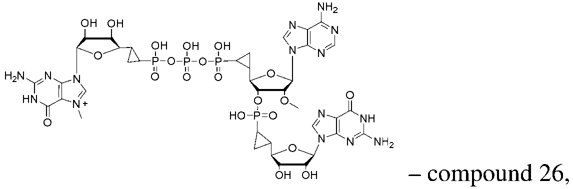

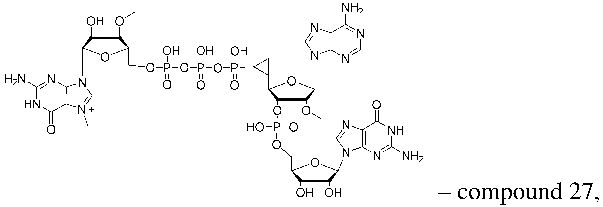

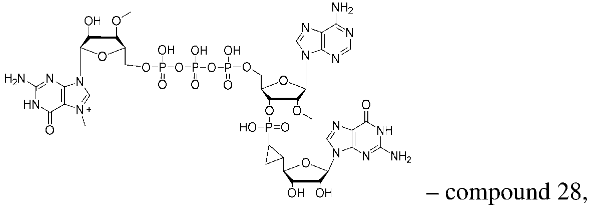

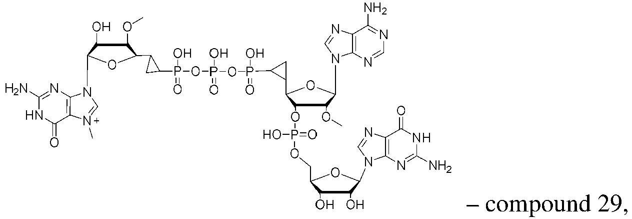

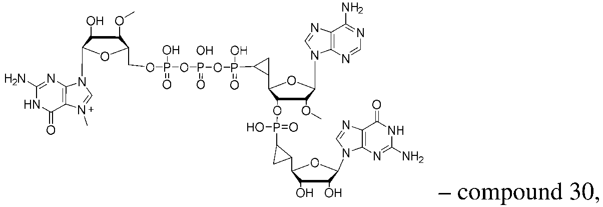

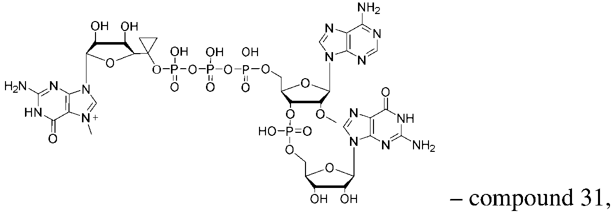

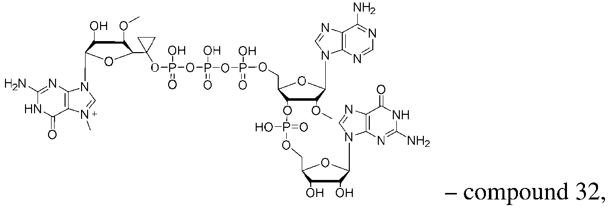

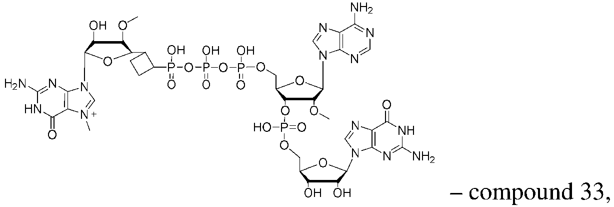

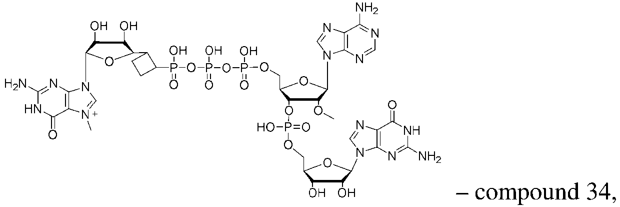

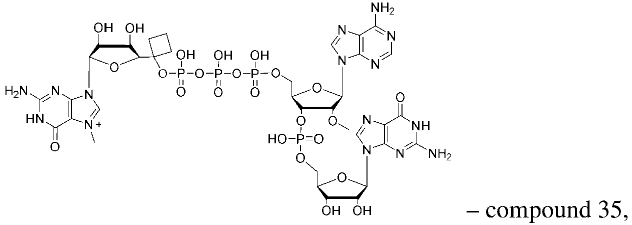

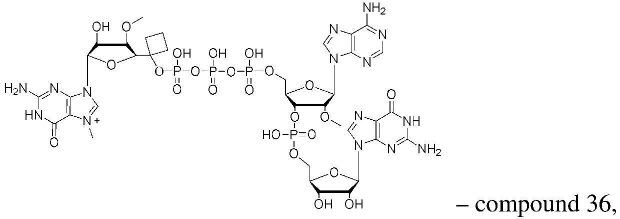

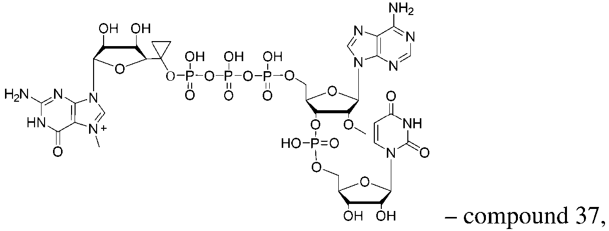

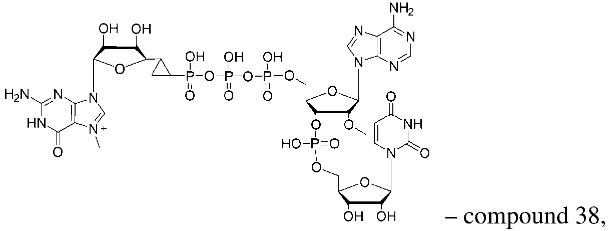

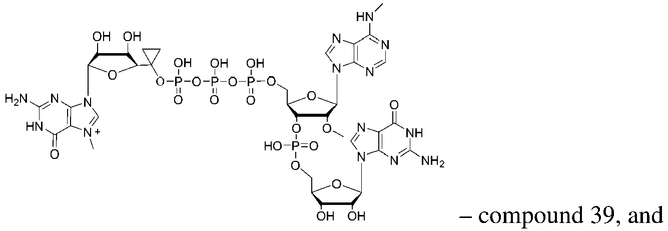

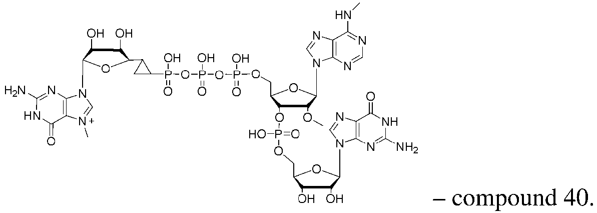

13. Use of the capping compound according to any one of claims 1-12 in the preparation of an *in vitro* co-transcriptional RNA capping reagent.

14. A complex, comprising the capping compound according to any one of claims 1-12 and a DNA template, wherein the DNA template comprises a promoter region comprising a transcription start site, the transcription start site having a first nucleotide at nucleotide position +1 and a second nucleotide at nucleotide position +2; and B₁ is complementary to a nucleobase at position +1 of the DNA template, and B₂ is complementary to a nucleobase at transcription template position +2 on the DNA template.

15. An RNA molecule, comprising the capping compound according to any one of claims 1-12.

16. A pharmaceutical composition, comprising the RNA molecule according to claim 15 and a pharmaceutically acceptable carrier.
